# EUROPEAN PATENT SPECIFICATION

(11) **EP 1 701 978 B1**
(45) Date of publication and mention of the grant of the patent: **11.02.2009**
(21) Application number: 04812804.5
(22) Date of filing: 02.12.2004
(51) Int. Cl.: C07K 14/705, G01N 33/68, A61K 49/00, A61K 38/00

(54) **Use of soluble T-Cadherin for the treatment of metabolic disorders**
Verwendung von löslichem T-Cadherin zur Behandlung von Stoffwechselkrankheiten
Utilisation de T-Cadhérine soluble pour le traitement de troubles métaboliques

(30) Priority: 03.12.2003 US 526956 P
(43) Date of publication of application: 20.09.2006
(73) Proprietor: WHITEHEAD INSTITUTE FOR BIOMEDICAL RESEARCH, Cambridge, MA 02142 (US)
(72) Inventor: HUG, Christopher, Brookline, MA 02446 (US); LODISH, Harvey, F., Brookline, MA 02146 (US)
(74) Representative: Merck Serono International S.A. Intellectual Property
(86) International application number: PCT/US2004/040363
(87) International publication number: WO 2005/057222

(56) References cited:
- WO-A-01/60853
- WO-A-02/23184
- WO-A-99/19477
- WO-A-02/053726
- WO-A-20/04096272
- WO-A-20/05049861
- US-B1- 6 358 920
- ROBINSON S W ET AL: "GENETIC MODELS OF OBESITY AND ENERGY BALANCE IN THE MOUSE" ANNUAL REVIEW OF GENETICS, ANNUAL REVIEWS INC., PALO ALTO, CA, US, vol. 34, 2000, pages 687-745, XP008017981 ISSN: 0066-4197
- DATABASE BIOSIS [Online] BIOSCIENCES INFORMATION SERVICE, PHILADELPHIA, PA, US; August 2003 (2003-08), TYRBERG B ET AL: "Diabetes development in T-cadherin deficient mice." XP008051535 Database accession no. PREV200300518094 & DIABETOLOGIA, vol. 46, no. Supplement 2, August 2003 (2003-08), page A30, 18TH CONGRESS OF THE INTERNATIONAL DIABETES FEDERATION; PARIS, FRANCE; AUGUST 24-29, 2003 ISSN: 0012-186X
- HUG CHRISTOPHER ET AL: "T-cadherin is a receptor for hexameric and high-molecular-weight forms of Acrp30/adiponectin" PROCEEDINGS OF THE NATIONAL ACADEMY OF SCIENCES OF THE UNITED STATES OF AMERICA, vol. 101, no. 28, 13 July 2004 (2004-07-13), pages 10308-10313, XP002342443 ISSN: 0027-8424

## Description

### Field of the Invention

This invention relates to the use of T-cadherin polypeptides for treating a metabolic disorder.

Metabolic disorders that may be treated using a modulator according to the present invention include, *e*.*g*., obesity, type II diabetes, insulin resistance, hypercholesterolemia, hyperlipidemia, dyslipidemia, syndrome X, anorexia and cachexia.

### Background of the Invention

### 1. T-cadherin

The cadherins comprise a large family of cell-surface proteins involved in calcium mediated cell-cell interactions and signaling. Cadherins exhibit cell-type and developmental specificity in expression and are aberrantly regulated in several human malignancies (see, *e.g.*, Conacci-Sorrell *et al.,* 2002).

T-cadherin, also known as H-cadherin or cadherin-13, is attached to the membrane via a GPI anchor at the C-terminus (Tanihara *et al.,* 1994). Compared to other members of the cadherin family, T-cadherin lacks the transmembrane domain and is released from the cell surface upon treatment of cells with phosphatidylinositol phospholipase-C. T-cadherin was initially described in the nervous system, but its tissue distribution is more widespread, with highest expression in the cardiovascular system and lower levels in muscle. In the vasculature, it is localized to the intima and media and is expressed on endothelial and smooth muscle cells (Ivanov *et al.,* 2001).

Two forms of T-cadherin are found on the cell surface. One of them, a 135 kDa polypeptide, is cleaved near the N-terminus to generate a second 105 kDa form (Tkachuk *et al*., 1998). Both forms of the protein are found on the cell surface, where they are capable of binding to LDL particles through association of the GPI anchor with lipoproteins (Niermann *et al.,* 2000).

Although no signaling pathways are known to involve T-cadherin, it has been suggested that T-cadherin may participate in signaling through its association with other membrane proteins and incorporation into specific lipid domains of the cell membrane (Doyle *et al.,* 1998). It has been shown that T-cadherin can regulate growth of cells in the nervous system (Takeuchi *et al.,* 2000). Furthermore, T-cadherin suppresses the growth of astrocytes and is reduced in a glioblastoma cell line (Huang *et al.,* 2003). It has also been suggested that T-cadherin may be involved in the control of the normal vascular architecture and its remodeling during atherogenesis (Ivanov *et al.,* 2001).

US 6358920 discloses a huge number of short peptides derived from more than 20 different cadherins. It only vaguely refers to the treatment of the metabolic disorders diabetes and obesity by modulating agents comprising an octapeptide representing a cell adhesion recognition amino acid sequence.

WO99/19477 teaches that the peptides, which are cleaved from Pro-cadherins while being processed into the corresponding cadherins, represent so-called "cadherin growth factor regions" (CGFRs) and that the activities of these CGFRs can be exploited therapeutically. In this context, it discloses the CGFR of T-cadherin for treatment of a metabolic disorder. This CGFR consists of the amino acids 23-138 of SEQ ID NO: 1.

WO2004/096272 discloses soluble forms of T-cadherin, wherein said soluble forms are polypeptides consisting of fragments of up to 118 amino acids of a polypeptide consisting of amino acids 23 to 692 of SEQ ID NO: 1. It also discloses medical uses of these soluble forms for metabolic disorders.

### 2. Obesity

### 2.1. Symptoms

Obesity is the accumulation of excessive body fat. The severity of obesity is determined by measuring height and weight. Often, these measurements are converted to body mass index (BMI): the weight in kilograms divided by height in meters squared. A value of 25 to 29.9 indicates overweight or mild obesity, and a value of 30 or higher indicates obesity and a need for treatment. In the United States, overweight and obesity now affect more than 50% of adults, reflecting a rapid increase in prevalence.

Obesity results from consuming more calories than the body uses. Genetic and environmental factors influence body weight, but precisely how they interact to determine a person's weight is still unclear. Recent research suggests that on the average, the genetic influence contributes to about 33 percent of body weight. An increase in the size or number of fat cells or both adds to the amount of fat stored in the body. Obese people, particularly those who became obese during childhood, may have up to five times more fat cells than people of normal weight. Because the number of cells cannot be reduced, weight can be lost only by reducing the amount of fat in each cell.

Accumulation of excess fat below the diaphragm and in the chest wall may put pressure on the lungs, causing difficulty in breathing and shortness of breath, even with minimal exertion. The difficulty in breathing may seriously interferes with sleep, causing momentary cessation of breathing (sleep apnea), leading to daytime sleepiness and other complications. Obesity may also cause various orthopedic problems, skin disorders and swelling of the feet and ankles. Severe complications of obesity include a much higher risk of coronary artery disorder and of its major risk factors type II diabetes, hyperlipidemia and hypertension. Much of the morbidity associated with obesity is associated with type II diabetes, as poorly controlled diabetes and obesity lead to a constellation of symptoms that are together known as syndrome X, or metabolic syndrome (see, *e.g*., Roth *et al.,* 2002).

### 2.2. Treatment

Most weight management programs are based on behavior modification. Dieting is usually considered less important than making permanent changes in eating and exercise habits. Increasingly, doctors are prescribing drugs such as orlistat or sibutramine to reduce body weight. Such a drug can reduce weight by about 10 percent within 6 months and maintains the loss as long as the drug is continued. However, when it's discontinued, the weight is promptly regained, and the long-term effects of current treatments for obesity are disappointing. Although considerable progress has been made in helping people lose weight, they usually regain it within 3 years. The many serious complications of obesity make treatment important, and treatment by surgery is becoming more and more common in case of severe obesity.

### 3. Diabetes

### 3.1. Symptoms

Diabetes is a disorder in which blood levels of glucose are abnormally high because the body does not release or use insulin adequately. Diabetes results when the body does not produce enough insulin to maintain normal blood sugar levels or when cells do not respond appropriately to insulin.

People with type I diabetes (insulin-dependent diabetes) produce little or no insulin at all. Although about 6 percent of the United States population has some form of diabetes, only about 10 percent of all diabetics have type I disorder. Most people who have type I diabetes developed the disorder before age 30. In type I diabetes more than 90 percent of the insulin-producing cells (beta cells) of the pancreas are permanently destroyed. The resulting insulin deficiency is severe, and to survive, a person with type I diabetes must regularly inject insulin.

In type II diabetes (non-insulin-dependent diabetes), the pancreas continues to manufacture insulin, sometimes even at higher than normal levels. However, the body develops resistance to its effects, resulting in a relative insulin deficiency. Type II diabetes may occur in children and adolescents but usually begins after age 30 and becomes progressively more common with age: About 15 percent of people over age 70 have type II diabetes. Obesity is a risk factor for type II diabetes, and 80 to 90 percent of the people with this disorder are obese.

Other less common causes of diabetes are abnormally high levels of corticosteroids, pregnancy (gestational diabetes), drugs, and poisons that interfere with the production or effects of insulin, resulting in high blood sugar levels.

People with type II diabetes may not have any symptoms for years or decades. When insulin deficiency progresses, symptoms may develop. Increased urination and thirst are mild at first and gradually worsen over weeks or months. If the blood sugar level becomes very high (often exceeding 1,000 mg/dl), the person may develop severe dehydration, which may lead to mental confusion, drowsiness, seizures, and nonketotic hyperglycemic-hyperosmolar coma.

Over time, elevated blood sugar levels damage blood vessels, nerves, and other internal structures. Complex sugar-based substances build up in the walls of small blood vessels, causing them to thicken and leak. As they thicken, they supply less and less blood, especially to the skin and nerves. Poorly controlled blood sugar levels also tend to cause the blood levels of fatty substances to rise, resulting in accelerated atherosclerosis (the buildup of plaque in blood vessels). Atherosclerosis is between two and six times more common in diabetics than in non-diabetics and occurs in both men and women. Poor circulation through both the large and small blood vessels can harm the heart, brain, legs, eyes, kidneys, nerves, and skin and makes healing injuries slow.

For all of these reasons, people with diabetes may experience many serious long-term complications. Heart attacks and strokes are more common. Damage to the blood vessels of the eye can cause loss of vision (diabetic retinopathy). The kidneys can malfunction, resulting in kidney failure that requires dialysis. Damage to nerves can manifest in several ways. If a single nerve malfunctions (mononeuropathy), an arm or leg may suddenly become weak. If the nerves to the hands, legs, and feet become damaged (diabetic polyneuropathy), sensation may become abnormal and tingling or burning pain and weakness in the arms and legs may develop. Damage to the nerves of the skin makes repeated injuries more likely because the person cannot sense changes in pressure or temperature. Poor blood supply to the skin can also lead to ulcers, and all wounds heal slowly. Foot ulcers may become so deep and infected and heal so poorly that part of the leg may need to be amputated.

### 3.2. Treatment

The main goal of diabetes treatment is to keep blood sugar levels within the normal range as much as possible. Completely normal levels are difficult to maintain, but the more closely they can be kept within the normal range, the less likely that temporary or long-term complications will develop. The main problem with trying to control blood sugar levels tightly is an increased chance of overshooting, resulting in low blood sugar levels (hypoglycemia).

The treatment of diabetes requires attention to weight control, exercise, and diet. Many obese people with type II diabetes would not need medication if they lost weight and exercised regularly. However, as discussed above, weight reduction is difficult. Therefore, either insulin replacement therapy or an oral hypoglycemic medication is often needed.

Oral hypoglycemic drugs such as glipizide, glyburide, tolbutamide, and chlorpropamide may lower blood sugar levels adequately in people with type II diabetes by stimulating the pancreas to release insulin and by increasing its effectiveness. Another type of oral drug, metformin, increases the body's response to its own insulin. Yet another drug, acarbose, works by delaying absorption of glucose in the intestine. If these oral hypoglycemic drugs cannot control blood sugar well enough, an insulin replacement therapy is needed.

An insulin replacement therapy can be accomplished only by daily injections, being thus a heavy treatment. New forms of insulin, such as a nasal spray, are being tested. To date, these new forms have not worked well because variability in the rate of absorption leads to problems in determining dose.

### 4. Acrp30

Adipose tissue, while long known for its capacity to store fat, has an important role as the source for a number of hormones and paracrine mediators, including resistin, adipsin, leptin, and TNF-α. Collectively, these molecules are termed adipokines, to emphasize their role as hormone and site of synthesis. Acrp30, also referred to as adiponectin or ApM-1, is one such adipokine and is produced exclusively by adipose tissue.

Mouse Acrp30 was first identified in, 1995 (Scherer *et al.,* 1995), and was shown to be up-regulated over 100-fold during adipocyte differentiation. The human homolog was identified in, 1996 (Maeda *et al.,* 1996). Acrp30 contains an amino-terminal signal sequence, followed by a central region comprising collagen repeats, and a carboxyl-terminal domain with homology to the globular complement factor C1q. As used herein, the term "Acrp30" refers both to the human and to the murine proteins, and to their homologs in any other species.

### 4.1. The role of Acrp 30 in obesity and type II diabetes

Several studies have demonstrated that Acrp30 is linked to obesity and type II diabetes. Genetic data have demonstrated that linkage of type II diabetes with non-coding Single Nucleotide Polymorphisms (SNPs) located within the Acrp30 gene in a Japanese cohort of patients (Hara *et al.,* 2002). It was further demonstrated that missense mutations affecting the globular head are correlated with serum levels of Acrp30 (Kondo *et al.,* 2002).

In addition, serum levels of Acrp30 are decreased in several models of obesity, including leptin-deficient mice, leptin-receptor deficient mice, and monkey models (see, *e.g.*, Hu *et al.,* 1996; Yamauchi *et al.,* 2001). In human studies, Acrp30 levels are inversely correlated to both diabetes and obesity, and they are further reduced in patients with coronary artery disorder (Arita *et al.,* 1999). Further evidence for a causal relationship between reduced levels of Acrp30 and development of insulin resistance and type II diabetes was obtained by Lindsay *et al.* (2002), who showed that individuals in the Pima Indian population who had lower serum levels of Acrp30 were more likely to develop type II diabetes than those with higher levels.

Gene disruption experiments yielded supporting evidence for the involvement of Acrp30 in obesity and type II diabetes. Maeda *et al.* (2002) found that homozygous Acrp30-deficient mice were not hyperglycemic when maintained on a normal diet, but they did exhibit reduced clearance of serum free fatty acid. When switched to a high-fat, high-sucrose diet, they exhibited severe insulin resistance and demonstrated increased weight gain relative to control animals.

In addition to its pivotal role in obesity and diabetes, Acrp30 has been shown to play a role in other disorders such as, *e.g.,* polycystic ovary syndrome (Panidis *et al.,* 2003), endometrial cancer (Petridou *et al., 2003),* preeclampsia (Ramsay *et al.,* 2003), fatty liver (Xu *et al.,* 2003) and nephrotic syndrome (Zoccali *et al.,* 2003). Acrp30 has also been shown to display anti-inflammatory properties (Yokota *et al.,* 2000).

In view of the above studies, exogenous Acrp30 is a promising therapeutics for treating various disorders including metabolic disorders such as obesity and type II diabetes, gynecologic disorders, liver disorders and chronic inflammatory disorders.

### 4.2. The different Acrp30 species

Acrp30 is present in serum at high concentration (5-10 µg/ml) and exists as multiple pools of different apparent molecular weight (Scherer *et a*/., 1995).

The structure of these species of different apparent molecular weight was investigated by Tsao *et al.* (2002, 2003). When expressed in bacteria as a full-length fusion protein and separated by gel-filtration chromatography, three species of Acrp30 were identified: hexamers and two species of trimers. Eukaryotic cell expression studies generated three Acrp30 species: a high-molecular weight (HMW) species, which is not seen in bacterially produced protein, and species corresponding to hexamers and one species of trimers.

Several studies have focused on polypeptides comprising the globular head of Acrp30, hereafter referred to as gAcrp30. There are differences between the activities of Acrp30 and gAcrp30 *in vivo,* although the two polypeptides share several similarities. Following long-term treatment with recombinant protein, there is a greater reduction in weight with injection of gAcrp30 compared to Acrp30, in spite of a lack of difference in food intake (Fruebis *et al.,* 2001). On the other hand, Acrp30 reduced glucose production synergistically with insulin, whereas gAcrp30 had no activity in this assay (Berg *et al.,* 2001).

To determine the potential activity of these different species, a number of transcriptional response elements driving expression of a luciferase reporter gene were assayed in C2C 12 myocytes, cells that are known to respond to Acrp30 (Tsao *et al.,* 2003). The NF-κB-responsive E-selectin promoter demonstrated increased activity in response to addition of Acrp30. Only the fractionated hexamer and HMW species were active, whereas both gAcrp30 and the trimer were inactive in this assay. IL-6, the expression of which is increased by NF-κB in C2C12 myotubes, is produced at high levels by skeletal muscle during exercise, and is thought to trigger increased fatty acid and glucose production from adipose tissue and liver, thus providing an increase in circulating fuel for use by muscle (Febbraio *et al.*, 2002; Kosmidou *et al.,* 2002). Thus hexameric and HMW species of Acpr30 may indirectly stimulate lipolysis from fat tissue through the NF-κB induced release of IL-6 from skeletal muscle (Tsao *et al.,* 2003).

Acrp30 being involved in obesity and type II diabetes, modulation of proteins of the Acrp30 signaling pathway is a treatment option for metabolic disorders such as obesity, type II diabetes, insulin resistance, hypercholesterolemia, hyperlipidemia, dyslipidemia, syndrome X, anorexia and cachexia. However, isolation of Acrp30 receptors has proven elusive. Binding of Acrp30 to collagens and to human aortic endothelial cells (HAECs) has been described *in vitro* (Okamoto *et al.,* 2000). Recently, two receptors for globular and full-length Acrp30 of unknown function, termed AdipoR1 and AdipoR2, have been described (Yamauchi *et al.,* 2003). These two Acrp30 receptors are predicted to contain seven transmembrane domains, but to be structurally and functionally distinct from G-protein-coupled receptors. However, receptors for the hexameric and HMW species of Acrp30 have not been described in scientific literature.

### Brief Summary of the Invention

The present invention stems from the finding of a novel Acrp30 receptor. Specifically, it has been shown in the frame of the present invention that T-cadherin is a receptor for the hexameric and high molecular weight (HMW) species of Acrp30.

Therefore, a first aspect of the specification relates to the use of a T-cadherin polypeptide as a target for screening candidate modulators for candidate drugs for the treatment of a disorder selected from the group consisting of a metabolic disorder, a gynecologic disorder, a chronic inflammatory disorder and a liver or renal disorder, wherein said candidate drug is a T-cadherin modulator.

A second aspect relates to the use of a modulator of a T-cadherin polypeptide for the preparation of a medicament for the treatment of a disorder selected from the group consisting of a metabolic disorder, a gynecologic disorder, a chronic inflammatory disorder and a liver or renal disorder.

A third aspect relates to the use of a T-cadherin polypeptide as a target for screening for natural binding partners, wherein said natural binding partner is a candidate drug for the treatment of a disorder selected from the group consisting of a metabolic disorder, a gynecologic disorder, a chronic inflammatory disorder and a liver or renal disorder.

A fourth aspect relates to the use of a soluble form of T-cadherin as medicament.

A fifth aspect, which represents the present invention, relates to the use of a soluble form of T-cadherin for the preparation of a medicament for the treatment of a metabolic disorder.

A sixth aspect relates to a method of assessing the efficiency of a modulator of a T-cadherin polypeptide for the treatment of obesity, said method comprising administering said modulator to an animal model for obesity, wherein a determination that said modulator ameliorates a representative characteristic of obesity in said animal model indicates that said modulator is a drug for the treatment of obesity.

A seventh aspect relates to a method of assessing the efficiency of a modulator of a T-cadherin polypeptide for the treatment of type II diabetes, said method comprising administering said modulator to an animal model for type II diabetes, wherein a determination that said modulator ameliorates a representative characteristic of type II diabetes in said animal model indicates that said modulator is a drug for the treatment of type II diabetes.

### Brief Description of the Drawings

**Figure 1** shows the result of Fluorescence-activated cell-sorter binding assays of C2C12, CHO and Ba/F3 cells, as explained in Example 2.
**Figure** 2 shows the result of Fluorescence-activated cell-sorter binding assays binding assay of control Ba/F3 cells and of T-cadherin expressing Ba/F3 cells, as explained in Example 4.2.
**Figure 3A** shows the ELISA analysis of binding of different species of Acrp30 to CHO-GFP cells, as explained in Example 4.3.
**Figure 3B** shows the ELISA analysis of binding of different species of Acrp30 to T-cadherin expressing CHO cells, as explained in Example 4.3.
**Figure 4** shows activation of NF-κB in C2C12 cells, as explained in Example 5.

### Brief Description of the Sequences

**SEQ ID NO: 1** corresponds to the human T-cadherin full-length precursor.
**SEQ ID NO: 2** corresponds to the human Acrp30 full-length precursor.
**SEQ ID NO: 3** corresponds to the murine Acrp30 full-length precursor.
**SEQ ID NO: 4** corresponds to the murine T-cadherin full-length precursor.
**SEQ ID NOs: 5-18** correspond to primer sequences.

### Detailed Disclosure of the Invention

The present invention stems from the finding of a novel Acrp30 receptor. Specifically, it has been shown in the frame of the present invention that T-cadherin is a receptor for the hexameric and high molecular weight (HMW) species of Acrp30. Data showing that T-cadherin over-expression suppresses Acrp30-induced NF-κB-mediated transcription are further provided. Accordingly, the present specification provides means to identify compounds useful in the treatment of metabolic disorders such as, *e.g*., obesity, type II diabetes, insulin resistance, hypercholesterolemia, hyperlipidemia, dyslipidemia, syndrome X, anorexia and cachexia. Specifically, the specification relates to the use of T-cadherin polypeptides as targets for screening for modulators thereof. The use of said modulators for treating metabolic disorders is a further aspect of the present specification.

A first aspect of the present specification is directed to the use of a T-cadherin polypeptide as a target for screening candidate modulators for candidate drugs for the treatment of a disorder selected from the group consisting of a metabolic disorder, a gynecologic disorder, a chronic inflammatory disorder and a liver or renal disorder, wherein said candidate drug is a T-cadherin modulator.

As used throughout the present specification, the term "T-cadherin polypeptide" refers both to full-length T-cadherin proteins and to biologically active fragments thereof. T-cadherin polypeptides may be of human or of animal origin. Preferably, T-cadherin polypeptides are of human origin.

Most preferably, the human T-cadherin polypeptide are selected from the group consisting of:
a) a polypeptide comprising SEQ ID NO: 1;
b) a polypeptide comprising amino acid 23 to 713 of SEQ ID NO: 1;
c) a polypeptide comprising amino acid 23 to 693 of SEQ ID NO: 1;
d) a polypeptide comprising amino acids 140 to 713 of SEQ ID NO: 1;
e) a polypeptide comprising amino acids 140 to 693 of SEQ ID NO: 1;
f) a mutein of any of (a) to (e), wherein the amino acid sequence has at least 95%, 96%, 97%, 98% or 99% identity to at least one of the sequences in (a) to (e);
g) a mutein of any of (a) to (e) which is encoded by a nucleic acid which hybridizes to the complement of a DNA sequence encoding any of (a) to (e) under highly stringent conditions; and
h) a mutein of any of (a) to (e) wherein any changes in the amino acid sequence are conservative amino acid substitutions of the amino acid sequences in (a) to (e).

The term "T-cadherin polypeptide" embraces naturally ocuring, recombinant, chimeric, synthetic and chemically synthetized polypeptides. The term "T-cadherin polypeptide" further embrace fragments of at least 50, 100, 150, 200, 250, 300, 350, 400, 450, 500, 550, 600 or 650 amino acids of T-cadherin. The term "T-cadherin polypeptide" further embrace muteins of T-cadherin. As used herein the term "muteins" refers to analogs of T-cadherin, in which one or more of the amino acid residues of a natural T-cadherin are replaced by different amino acid residues, or are deleted, or one or more amino acid residues are added to the natural sequence of T-cadherin, without lowering considerably the activity of the resulting products as compared with the wild-type T-cadherin: These muteins are prepared by known synthesis and/or by site-directed mutagenesis techniques, or any other known technique suitable therefore.

Muteins of T-cadherin that can be used in accordance with the present specification include a finite set of substantially corresponding sequences as substitution peptides or polynucleotides which can be routinely obtained by one of ordinary skill in the art, without undue experimentation, based on the teachings and guidance presented herein and known in the art.

T-cadherin polypeptides in accordance with the present specification include proteins encoded by a nucleic acid, such as DNA or RNA, which hybridizes to DNA or RNA, which encodes T-cadherin, in accordance with the present invention, under moderately or highly stringent conditions. The term "stringent conditions" refers to hybridization and subsequent washing conditions, which those of ordinary skill in the art conventionally refer to as "stringent" (see, *e.g*., Sambrook *et al.,* 1989).

Without limitation, examples of stringent conditions include washing conditions 12-20°C below the calculated Tm of the hybrid under study in, *e.g*., 2 x SSC and 0.5% SDS for 5 minutes, 2 x SSC and 0.1% SDS for 15 minutes; 0.1 x SSC and 0.5% SDS at 37°C for 30-60 minutes and then, a 0.1 x SSC and 0.5% SDS at 68°C for 30-60 minutes. Those of ordinary skill in this art understand that stringency conditions also depend on the length of the DNA sequences, oligonucleotide probes (such as 10-40 bases) or mixed oligonucleotide probes. If mixed probes are used, it is preferable to use tetramethyl ammonium chloride (TMAC) instead of SSC.

The T-cadherin polypeptides that can be used in accordance with the present specification include muteins having an amino acid sequence at least 50% identical, more preferably at least 60% identical, and still more preferably 70%, 80%, 90%, 95%, 96%, 97%, 98% or 99% identical to a T-cadherin of SEQ ID NO: 1 or SEQ ID NO: 4 or to a fragment thereof. By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% (5 of 100) of the amino acid residues in the subject sequence may be inserted, deleted, or substituted with another amino acid.

For sequences where there is not an exact correspondence, a "% identity" may be determined. In general, the two sequences to be compared are aligned to give a maximum correlation between the sequences. This may include inserting "gaps" in either one or both sequences, to enhance the degree of alignment. A % identity may be determined over the whole length of each of the sequences being compared (so-called global alignment), that is particularly suitable for sequences of the same or very similar length, or over shorter, defined lengths (so-called local alignment), that is more suitable for sequences of unequal length.

Preferred changes for muteins that can be used in accordance with the present specification are what are known as "conservative" substitutions. Conservative amino acid substitutions of T-cadherin polypeptides, may include synonymous amino acids within a group which have sufficiently similar physicochemical properties that substitution between members of the group will preserve the biological function of the molecule (Grantham, 1974). It is clear that insertions and deletions of amino acids may also be made in the above-defined sequences without altering their function, particularly if the insertions or deletions only involve a few amino acids, *e.g*. under thirty, and preferably under ten, and do not remove or displace amino acids which are critical to a functional conformation, *e.g*. cysteine residues. Proteins and muteins produced by such deletions and/or insertions come within the purview of the present specification.

Preferably, the synonymous amino acid groups are those defined in Table I. More preferably, the synonymous amino acid groups are those defined in Table II; and most preferably the synonymous amino acid groups are those defined in Table III.

**TABLE I**

| **Preferred Groups of Synonymous Amino Acids** | |
|---|---|
| *Amino Acid* | *Synonymous Group* |
| Ser | Ser, Thr, Gly, Asn |
| Arg | Arg, Gln, Lys, Glu, His |
| Leu | Ile, Phe, Tyr, Met, Val, Leu |
| Pro | Gly, Ala, Thr, Pro |
| Thr | Pro, Ser, Ala, Gly, His, Gln, Thr |
| Ala | Gly, Thr, Pro, Ala |
| Val | Met, Tyr, Phe, Ile, Leu, Val |
| Gly | Ala, Thr, Pro, Ser, Gly |
| Ile | Met, Tyr, Phe, Val, Leu, Ile |
| Phe | Trp, Met, Tyr, Ile, Val, Leu, Phe |
| Tyr | Trp, Met, Phe, Ile, Val, Leu, Tyr |
| Cys | Ser, Thr, Cys |
| His | Glu, Lys, Gln, Thr, Arg, His |
| Gln | Glu, Lys, Asn, His, Thr, Arg, Gln |
| Asn | Gln, Asp, Ser, Asn |
| Lys | Glu, Gln, His, Arg, Lys |
| Asp | Glu, Asn, Asp |
| Glu | Asp, Lys, Asn, Gln, His, Arg, Glu |
| Met | Phe, Ile, Val, Leu, Met |
| Trp | Trp |

**TABLE II**

| **More Preferred Groups of Synonymous Amino Acids** | |
|---|---|
| *Amino Acid* | *Synonymous Group* |
| Ser | Ser |
| Arg | His, Lys, Arg |
| Leu | Leu, Ile, Phe, Met |
| Pro | Ala, Pro |
| Thr | Thr |
| Ala | Pro, Ala |
| Val | Val, Met, Ile |
| Gly | Gly |
| Ile | Ile, Met, Phe, Val, Leu |
| Phe | Met, Tyr, Ile, Leu, Phe |
| Tyr | Phe, Tyr |
| Cys | Cys, Ser |
| His | His, Gln, Arg |
| Gln | Glu, Gln, His |
| Asn | Asp, Asn |
| Lys | Lys, Arg |
| Asp | Asp, Asn |
| Glu | Glu, Gln |
| Met | Met, Phe, Ile, Val, Leu |
| Trp | Trp |

**TABLE III**

| **Most Preferred Groups of Synonymous Amino Acids** | |
|---|---|
| *Amino Acid* | *Synonymous Group* |
| Ser | Ser |
| Arg | Arg |
| Leu- | Leu, Ile, Met |
| Pro | Pro |
| Thr | Thr |
| Ala | Ala |
| Val | Val |
| Gly | Gly |
| Ile | Ile, Met, Leu |
| Phe | Phe |
| Tyr | Tyr |
| Cys | Cys, Ser |
| His | His |
| Gln | Gln |
| Asn | Asn |
| Lys | Lys |
| Asp | Asp |
| Glu | Glu |
| Met | Met, Ile, Leu |
| Trp | Trp |

Examples of production of amino acid substitutions in proteins which can be used for obtaining muteins of T-cadherin, polypeptides for use in the present invention include any known method steps, such as presented in US patents 4,959,314, 4,588,585 and 4,737,462, to Mark et al.; 5,116,943 to Koths *et al.,* 4,965,195 to Namen *et al.;* 4,879,111 to Chong *et al.;* and 5,017,691 to Lee et al.*;* and lysine substituted proteins presented in US patent No. 4,904,584 (Shaw et al.*).*

Preferably, the muteins of the present specification exhibit substantially the same biological activity as the T-cadherin polypeptide to which it corresponds. Most preferably, the muteins of the present invention exhibit enhanced biological activity as the T-cadherin polypeptide to which it corresponds.

As used herein, the term "modulator" refers to a compound that increases or decreases the activity of a T-cadherin polypeptide. As used herein, a "T-cadherin modulator" refers to a compound that increases or decreases the activity of a T-cadherin polypeptide and/or to a compound that increases or decreases the transcription level of the T-cadherin mRNA. The term "modulator" encompasses both agonists and antagonists.

As used herein, a "T-cadherin agonist" refers to a compound that has an effect in the same direction on T-cadherin activity as Acrp30. A compound having "an effect in the same direction" on T-cadherin activity as Acrp30 refers to a compound that (i) when tested in an assay wherein Acrp30 enhances T-cadherin activity, said compound enhances T-caherin activity; and/or (i) when tested in an assay wherein Acrp30 decreases T-cadherin activity, said compound decreases T-caherin activity. The terms "agonist" and "activator" are considered to be synonymous and can be used interchangeably throughout the present disclosure.

As used herein, a "T-cadherin antagonist" refers to a compound that has an opposite effect on T-cadherin activity compared to Acrp30. A compound having "an opposite effect" on T-cadherin activity as Acrp30 refers to a compound that (i) when tested in an assay wherein Acrp30 enhances T-cadherin activity, said compound decreases T-caherin activity; and/or (i) when tested in an assay wherein Acrp30 decreases T-cadherin activity, said compound increases T-caherin activity. The terms "antagonist" and "inhibitors" are considered to be synonymous and can be used interchangeably throughout the disclosure.

Methods for determining whether a modulator has an effect in the same direction or an opposite effect on T-cadherin activity as Acrp30 are further detailed below.

Methods that can be used for testing modulators for their ability to increase or decrease the activity of a T-cadherin polypeptide or to increase or decrease the expression of a T-cadherin mRNA are well known in the art and further detailed below. These assays can be performed either *in vitro or in vivo.*

Candidate modulators according to the present specification include naturally occurring and synthetic compounds. Such compounds include, *e.g.*, natural ligands, small molecules, aptamers, antisense mRNAs, small interfering RNAs, soluble forms of T-cadherin and antibodies. Such compounds are referred to as "candidate compounds" or to as "candidate modulators" in the present specification. Preferred candidate agonists include natural ligands, small molecules and aptamers. Preferred candidate antagonists include natural ligands, small molecules, aptamers, antisense mRNAs, small interfering RNAs, soluble forms of T-cadherin and antibodies. Most preferred candidate antagonists are soluble forms of T-cadherin.

As used herein, the term "natural ligand" refers to any signaling molecule that binds to a T-cadherin *in vivo* and includes molecules such as, *e.g.*, lipids, nucleotides, polynucleotides, amino acids, peptides, polypeptides, proteins, carbohydrates and inorganic molecules.

As used herein, the term "small molecule" refers to an organic molecule of relatively low weight. Preferably, a small molecule comprises less than 200 atoms. Most preferably, a small molecule comprises between about 50 atoms and 80 atoms.

As used herein, the term "antibody" refers to a protein produced by cells of the immune system or to a fragment thereof that binds to an antigen. The antibodies according to the specification may be polyclonal or monoclonal, chimeric, humanized, or even fully human. Recombinant antibodies and fragments thereof are characterized by high affinity binding to T-cadherin polypeptides *in vivo* and low toxicity. The antibodies which can be used in the specification are characterized by their ability to treat patients for a period sufficient to have good to excellent regression or alleviation of the pathogenic condition or any symptom or group of symptoms related to a pathogenic condition, and a low toxicity. Neutralizing antibodies are readily raised in animals such as rabbits, goat or mice by immunization with T-cadherin polypeptides. Immunized mice are particularly useful for providing sources of B cells for the manufacture of hybridomas, which in turn are cultured to produce large quantities of anti-T-cadherin monoclonal antibodies. Chimeric antibodies are immunoglobulin molecules characterized by two or more segments or portions derived from different animal species. Generally, the variable region of the chimeric antibody is derived from a non-human mammalian antibody, such as murine monoclonal antibody, and the immunoglobulin constant region is derived from a human immunoglobulin molecule. Preferably, both regions and the combination have low immunogenicity as routinely determined (Elliott et *al.,* 1994). Humanized antibodies are immunoglobulin molecules created by genetic engineering techniques in which the murine constant regions are replaced with human counterparts while retaining the murine antigen binding regions. The resulting mouse-human chimeric antibody preferably have reduced immunogenicity and improved pharmacokinetics in humans (Knight *et al.,* 1993). The antibody may be fully human. The technology for producing human antibodies is described in detail *e.g*. in WO 00/76310, WO 99/53049, US 6,162,963 and AU 5,336,100. One method for the preparation of fully human antibodies consist of "humanization" of the mouse humoral immune system, i.e. production of mouse strains able to produce human Ig (Xenomice), by the introduction of human immunoglobulin (Ig) loci into mice in which the endogenous Ig genes have been inactivated. The Ig loci are complex in terms of both their physical structure and the gene rearrangement and expression processes required to ultimately produce a broad immune response. Antibody diversity is primarily generated by combinatorial rearrangement between different V, D, and J genes present in the Ig loci. These loci also contain the interspersed regulatory elements, which control antibody expression, allelic exclusion, class switching and affinity maturation. Introduction of un-rearranged human Ig transgenes into mice has demonstrated that the mouse recombination machinery is compatible with human genes. Furthermore, hybridomas secreting antigen specific hu-mAbs of various isotypes can be obtained by Xenomice immunisation with antigen. Fully human antibodies and methods for their production are known in the art (see, *e.g*., WO 98/24893).

As used herein, the term "antisense mRNA" refers to a RNA molecule complementary to the strand normally processed into mRNA and translated, or to a RNA molecule complementary to a region thereof.

As used herein, the term "aptamer" refers to an artificial nucleic acid ligand (see, *e*.*g*., Ellington and Szostak, 1990).

As used herein, the term "small interfering RNA" refers to a double-stranded RNA inducing sequence-specific posttranscriptional gene silencing (see, *e*.*g*., Elbashir *et al.,* 2001).

As used herein, the term "soluble form of T-cadherin" refers to a T-cadherin polypeptide that is not attached to the membrane. T-cadherin polypeptides that are not attached to the membrane can easily be generated by those of skill in the art by mutating the GPI-anchor site of a T-cadherin polypeptide. For example, the glycine at position 693 of SEQ ID NO: 1 may be changed to another amino acid. Alternatively, the soluble form may be a fragment of T-cadherin lacking the GPI-anchor site. The soluble form of T-cadherin according to the present invention is selected from the group consisting of:
a) a polypeptide consisting of amino acids 23 to 692 of SEQ ID NO: 1;
b) a polypeptide consisting of a fragment of at least 250, 300, 350, 400, 450, 500, 550, 600 or 650 amino acids of (a);
c) a mutein of any of (a) or (b), wherein the amino acid sequence has at least 90% 95%, 96%, 97%, 98% or 99% identity to at least one of the sequences in (a) or (b);
d) a mutein of any of (a) or (b) which is encoded by a nucleic acid which hybridizes to the complement of a DNA sequence encoding any of (a) or (b) under highly stringent conditions; and
e) a mutein of any of (a) or (b) wherein any changes in the amino acid sequence are conservative amino acid substitutions of the amino acid sequences in (a) or (b).
Preferably, said soluble form of T-cadherin binds Acrp30. Preferably, said soluble form of T-cadherin binds hexameric and/or HMW species of Acrp30.

As used herein, the term "HMW species of Acrp30" refers to a complex of Acrp30 polypeptides comprising more than six Acrp30 polypeptides. The apparent molecular mass of murine HMW species of Acrp30 is of about 630 kDa. As used herein, the term "hexameric species of Acrp30" refers to a complex of six Acrp30 polypeptides. The apparent molecular mass of murine hexameric species is of about 410 kDa. Such multimeric or hexameric complexes can be purified and/or separated by, *e.g*., gel filtration as described in Tsao *et al.* (2002).

Soluble forms of T-cadherin in accordance with the present specification further include chimeric T-cadherin polypeptide comprising T-cadherin or a fragment thereof fused to a heterologous polypeptide.

In accordance with the present specification, said soluble form of T-cadherin comprises T-cadherin or a fragment thereof fused to all or a portion of an immunoglobulin. Methods for making immunoglobulin fusion proteins are well known in the art, such as the ones described in WO 01/03737, for example. The person skilled in the art will understand that the resulting fusion protein of the invention retains the biological activity of T-cadherin, in particular the binding to hexameric and/or HMW species of Acrp30. The fusion may be direct, or via a short linker peptide which can be as short as 1 to 3 amino acid residues in length or longer, for example, 13 amino acid residues in length. Said linker may be a tripeptide of the sequence E-F-M (Glu-Phe-Met), for example, or a 13-amino acid linker sequence comprising Glu-Phe-Gly-Ala-Gly-Leu-Val-Leu-Gly-Gly-Gln-Phe-Met introduced between the T-cadherin sequence and the immunoglobulin sequence. The resulting fusion protein has improved properties, such as an extended residence time in body fluids (half-life), increased specific activity, increased expression level, or the purification of the fusion protein is facilitated. Preferably, T-cadherin or a fragment thereof is fused to the constant region of an Ig molecule. Preferably, it is fused to heavy chain regions, like the CH2 and CH3 domains of human IgG1, for example. Other isofonns of Ig molecules are also suitable for the generation of fusion proteins according to the present specification, such as isoforms IgG2 or IgG4, or other Ig classes, like IgM or IgA, for example. Fusion proteins may be monomeric or multimeric, hetero- or homomultimeric.

The soluble form of T-cadherin can be a chimeric molecule linking a soluble region of T-cadherin and all or a portion of Acrp30. Such chimera have been described for IL-6 and its receptor IL-6R (Chebath *et al.,* 1997, WO 99/02552 and WO 97/32891). IL-6R/IL-6 chimera binds with a higher efficiency to their cellular receptor *in vitro* than does the mixture of IL-6 with a soluble IL-6R (Kollet *et al.,* 1999). T-cadherin may for example be fused to full-length Acrp30. Alternatively, T-cadherin may be fused to the central region of Acrp30 that comprises collagen repeats. Such chimeras are expected to form hexameric and HMW soluble chimeric molecules.

The candidate drugs can be obtained using any of the numerous approaches in combinatorial library methods known in the art, including, *e.g*., biological libraries, spatially addressable parallel solid phase or solution phase libraries, and synthetic library methods using affinity chromatography selection. The biological library approach is generally used with peptide libraries, while the other four approaches are applicable to peptide, non-peptide oligomers, aptamers or small molecule libraries of compounds.

One example of a method that may be used for screening candidate compounds for a modulator is a method comprising the steps of:
a) contacting a T-cadherin polypeptide with the candidate compound; and
b) testing the activity of said T-cadherin polypeptide in the presence of said candidate compound;
wherein a difference or change in the activity of said T-cadherin polypeptide in the presence of said compound indicates that the compound is a modulator of said T-cadherin polypeptide. Preferably, such a method additionally comprises the step of comparing the activity of said T-cadherin polypeptide in the presence of said compound to the activity of said T-cadherin polypeptide in the absence of said compound. Also preferably, such a method also comprises the step of testing the activity of said T-cadherin polypeptide in the absence of said candidate compound.

Alternatively, the assay may be a cell-based assay comprising the steps of:
a) contacting a cell expressing a T-cadherin polypeptide with the candidate compound; and
b) testing the activity of said T-cadherin polypeptide in the presence of said candidate compound;
wherein a difference or a change in the activity of said T-cadherin polypeptide in the presence of said compound indicates that the compound is a modulator of said T-cadherin polypeptide. Preferably, such a method additionally comprises the step of comparing the activity of said T-cadherin polypeptide in the presence of said compound to the activity of said T-cadherin polypeptide in the absence of said compound. Also preferably, such a method also comprises the step of testing the activity of said T-cadherin polypeptide in the absence of said candidate compound.

The modulator may be an inhibitor or an activator. An inhibitor may decrease T-cadherin activity by, *e.g.*, 10%, 20%, 30%, 40%, 50%, 60%, 70%, 80%, 90%, 95% or 100% compared to T-cadherin activity in the absence of said inhibitor. An activator may increase T-cadherin activity by, *e.g.*, 10%, 20%, 30%, 40%, 50%, 60%, 70%, 80%, 90%, 95% or 100% compared to T-cadherin activity in the absence of said activator.

The modulator may modulate any activity of said T-cadherin polypeptide. The modulator may for example modulate T-cadherin mRNA expression within a cell, modulate the binding of the T-cadherin polypeptide to a natural binding partner such as, *e.g*., Acrp30, or modulate cell growth.

The activity of a T-cadherin polypeptide can be assessed by measuring binding of said T-cadherin polypeptide to Acrp30. Assays for measuring binding of a T-cadherin polypeptide to Acrp30 are known by those of skill the art. For example, the FACS assay described in Example 4.2. or the ELISA assay described in Example 4.3. of the present specification may be used. Such an assay ay for example comprise the steps of:
a) contacting a cell expressing a T-cadherin polypeptide with the candidate compound and with Acrp30; and
b) testing the binding of said T-cadherin polypeptide to Acrp30 in the presence of said candidate compound;
   wherein a difference or change in the binding of said T-cadherin polypeptide to Acrp30 in the presence of said compound indicates that the compound is a modulator of said T-cadherin polypeptide. Preferably, such a method additionally comprises the step of comparing the binding of said T-cadherin polypeptide to Acrp30 in the presence of said compound to the binding of said T-cadherin polypeptide to Acrp30 in the absence of said compound. Also preferably, such a method also comprises the steps of:
c) contacting a cell expressing a T-cadherin polypeptide with Acrp30; and
d) testing the binding of said T-cadherin polypeptide to Acrp30 in the absence of said candidate compound.

Acrp30 can be a hexameric species of Acrp30. Alternatively, Acrp30 can be a HMW species of Acrp30.

The activity of a T-cadherin polypeptide can also be assessed by measuring the T-cadherin mRNA levels within a cell. The activity can for example be measured using Northern blots, RT-PCR, quantitative RT-PCR with primers and probes specific for T-cadherin mRNAs. Alternatively, the expression of the T-cadherin mRNA is measured at the polypeptide level, by using labeled antibodies that specifically bind to the T-cadherin polypeptide in immunoassays such as ELISA assays, or RIA assays, Western blots or immunohistochemical assays.

Alternatively, the activity of a T-cadherin polypeptide can be assessed by measuring regulation of cell growth. Preferred cells are cells in the nervous system such as astrocytes. Other preferred cells are C2C12 myotubes. Such assays may for example be performed as described in Takeuchi *et al.* (2000) or in Huang *et al.* (2003).

As used throughout the present specification, the term "metabolic disorder" includes obesity, type II diabetes, insulin resistance, hypercholesterolemia, hyperlipidemia, dyslipidemia, syndrome X, atherosclerosis, anorexia and cachexia. The terms "obesity". "type II diabetes", "insulin resistance", "hypercholesterolemia", "hyperlipidemia", "dyslipidemia" and "atherosclerosis" refer to conditions defined in "The Merck Manual-Second Home Edition" (Publisher: Merck & Co). The term "syndrome X" refers to a constellation of atherosclerotic risk factors, including insulin resistance, hyperinsulinemia, dyslipidemia, hypertension and obesity (see, *e.g*., Roth *et al.,* 2002). The term "cachexia" refers to a condition characterised by loss of fat. As used herein, the term "cachexia" includes wasting related to AIDS and cancer. The term "anorexia" refers to a condition characterized by a distorted body image, fear of obesity and inability to maintain a minimally normal body weight.

As used throughout the present specification, the term "gynecologic disorder" refers to disorders that affect the female reproductive system and/or disorders linked with pregnancy. Such disorders include, *e.g.*, polycystic ovary syndrome, endometrial cancer, breast cancer, preeclampsia and eclampsia. As used throughout the present specification, the term "liver or renal disorder" includes, *e.g*., fatty liver, nephrotic syndrome and chronic renal syndrome. As used throughout the present specification, the term "chronic inflammatory disorder" refers to a chronic pathologic inflammation of a tissue or an organ of an individual. Chronic inflammatory diseases include, *e.g.*, psoriasis, psoriatic arthritis, rheumatoid arthritis, asthma, inflammatory bowel disorder and multiple sclerosis. All the disorders mentioned above refer to disorders defined in "The Merck Manual--Second Home Edition" (Publisher: Merck & Co). Other disorders that may be treated using the modulators and the soluble forms of T-cadherin of the present specification include any cancer associated with obesity and/or insulin resistance such as, *e*.*g*., endometrial cancer.

According to the present invention , the disorder is a metabolic disorder.

In a further preferred embodiment, the disorder is a metabolic disorder selected from the group consisting of obesity, type II diabetes, insulin resistance, hypercholesterolemia, hyperlipidemia, dyslipidemia and syndrome X. A T-cadherin polypeptide can be used as a target for screening candidate modulators for candidate drugs for the treatment of obesity, wherein said candidate drug is a T-cadherin modulator. Alternatively a T-cadherin polypeptide can be used as a target for screening candidate modulators for candidate drugs for the treatment of type II diabetes, wherein said candidate drug is a T-cadherin modulator. In addition, a T-cadherin polypeptide can be used as a target for screening candidate modulators for candidate drugs for the treatment of syndrome X, wherein said candidate drug is a T-cadherin modulator.

Preferred candidate drugs for the treatment of a disorder selected from the group consisting of obesity, type II diabetes, insulin resistance, hypercholesterolemia, hyperlipidemia, dyslipidemia and syndrome X are T-cadherin agonists. Thus a T-cadherin polypeptide can be used as a target for screening candidate compounds for candidate drugs for the treatment of a disorder selected from the group consisting of obesity, type II diabetes, insulin resistance, hypercholesterolemia, hyperlipidemia, dyslipidemia and syndrome X, wherein said candidate drug is a T-cadlerin agonist. Preferably, said candidate compounds are selected from the group consisting of natural ligands, small molecules and aptamers.

Determining whether a T-cadherin modulator is a T-cadherin agonist or a T-cadherin antagonist may for example be assessed using the assay described in Example 5. Such an assay may for example comprise the steps of:
a) contacting a cell expressing a T-cadherin polypeptide with a modulator; and
b) testing NF-κB-mediated transcription in the presence of said modulator;
   wherein a determination that said modulator has an effect in the same direction on NF-κB-mediated transcription as Acrp30 indicates that the compound is an agonist of said T-cadherin polypeptide, and wherein a determination that said modulator has an opposite effect on NF-κB-mediated transcription compared to Acrp30 indicates that the compound is an antagonist of said T-cadherin polypeptide. Preferably, such a method additionally comprises the steps of:
c) contacting a cell expressing a T-cadherin polypeptide with Acrp30; and
d) testing NF-κB-mediated transcription in the presence of Acrp30 and in the absence of said modulator.

In such an assay, Acrp30 is preferably a hexameric species or a HMW species of Acrp30. In such an assay, the cell expressing a T-cadherin polypeptide may be, *e*.*g*., a G2C12 cell line or a C2C12 cell overexpressing T-cadherin. Such an assay can also be performed with candidate compounds in order to screen for T-cadherin agonists or T-cadherin antagonists.

IL-6, which is produced at high levels by skeletal muscle during exercise, triggers increased fatty acid and glucose production from adipose tissue. Acrp30 is thought to play a role in the release of IL-6 from skeletal muscle through NF-κB activation (see, *e.g.*, Tsao *et al.,* 2003). Accordingly, determining whether a T-cadherin modulator is a T-cadherin agonist or a T-cadherin antagonist may be assessed by measuring IL-6 gene expression and/or IL-6 release from skeletal muscle cells. Such an assay may for example comprise the steps of:
a) contacting a cell expressing a T-cadherin polypeptide with a modulator; and
b) testing IL-6 gene expression and/or IL-6 release from skeletal muscle cells in the presence of said modulator;
   wherein a determination that said modulator has an effect in the same direction on IL-6 gene expression and/or IL-6 release from skeletal muscle cells as Acrp30 indicates that the compound is an agonist of said T-cadherin polypeptide, and wherein a determination that said modulator has an opposite effect on IL-6 gene expression and/or IL-6 release from skeletal muscle cells compared to Acrp30 indicates that the compound is an antagonist of said T-cadherin polypeptide. Preferably, such a method additionally comprises the steps of:
c) contacting a cell expressing a T-cadherin polypeptide with Acrp30; and
d) testing IL-6 gene expression and/or IL-6 release from skeletal muscle cells in the presence of Acrp30 and in the absence of said modulator.
In such an assay, Acrp30 is preferably a hexameric species or a HMW species of Acrp30.

Alternatively, determining whether a T-cadherin modulator is a T-cadherin agonist or a T-cadherin antagonist may be assessed by any of the known *in vivo* assays for assessing Acrp30 activity.

The disorder can be anorexia or cachexia. Thus a T-cadherin polypeptide can be used as a target for screening candidate modulators for candidate drugs for the treatment of anorexia or cachexia, wherein said candidate drug is a T-cadherin modulator. Preferred candidate drugs for the treatment of cachexia or anorexia are T-cadherin antagonists.

Alternatively, the disorder can be a gynecologic disorder, a chronic inflammatory disorder or a liver or renal disorder. Preferred candidate drugs for the treatment of cachexia or anorexia are T-cadherin agonists.

A further aspect of the present specification is directed to the use of a modulator of a T-cadherin polypeptide for preparing a medicament for the treatment of a disorder selected from the group consisting of a metabolic disorder, a gynecologic disorder, a chronic inflammatory disorder and a liver or renal disorder,. Preferably, said disorder is a metabolic disorder. Such a medicament comprises said modulator of a T-cadherin polypeptide in combination with any physiologically acceptable carrier. Physiologically acceptable carriers can be prepared by any method known by those skilled in the art. Physiologically acceptable carriers include but are not limited to those described in Remington's Pharmaceutical Sciences (Mack Publishing Company, Easton, USA, 1985). Pharmaceutical compositions comprising a modulator of a T-cadherin polypeptide and a physiologically acceptable carrier can be for, *e.g.*, intravenous, topical, rectal, local, inhalant, subcutaneous, intradermal, intramuscular, oral, intracerebral and intrathecal use. The compositions can be in liquid (*e.g.*, solutions, suspensions), solid (*e.g*., pills, tablets, suppositories) or semisolid (*e*.*g*., creams, gels) form. Dosages to be administered depend on individual needs, on the desired effect and the chosen route of administration.

Such a medicament comprising a T-cadherin modulator may be used in combination with any known drug for the treatment of said disorder. For example, when treating obesity, the modulator may be administered in combination with Orlistat, Sibutramine, Rimonabant, Axokine, Fluasterone and/or Famoxin. When treating type II diabetes, the modulator may for example be administered in combination with Acarbose, Acetohexamide, Chlorpropamide, Glimepiride, Glipizide, Glyburide, Metformin, Tolazamide, Tolbutamide, Nateglinide, Insulin, Insulin Aspart, Insulin glargine, Miglitol, V-411, Repaglinide, Rosiglitazone and/or Pioglitazone. Medicaments comprising a T-cadherin modulator may also be administered in the frame of a diet.

A modulator of a T-cadherin polypeptide can be used for preparing a medicament for the treatment of a metabolic disorder selected from the group consisting of obesity, type II diabetes, insulin resistance, hypercholesterolemia, hyperlipidemia, dyslipidemia and syndrome X. Said modulator is preferably an agonist.

Alternatively, a modulator of a T-cadherin polypeptide can be used for preparing a medicament for the treatment of cachexia or anorexia. Said modulator is preferably an antagonist.

In addition, a modulator of a T-cadherin polypeptide can be used for preparing a medicament for the treatment of a gynecologic disorder, a chronic inflammatory disorder or a liver or renal disorder. Said modulator is preferably an agonist.

A further aspect of the present specification is directed to the use of a T-cadherin polypeptide as a target for screening for natural binding partners, wherein said binding partner is a candidate drug for the treatment of a disorder selected from the group consisting of a metabolic disorder, a gynecologic disorder, a chronic inflammatory disorder and a liver or renal disorder. Said disorder is preferably a metabolic disorder. Said metabolic disorder is preferably selected from the group consisting of obesity, type II diabetes, insulin resistance, hypercholesterolemia, hyperlipidemia, dyslipidemia, syndrome X, anorexia and cachexia. Using a T-cadherin polypeptide as a target has a great utility for the identification of proteins involved in a metabolic disorder, and for providing new intervention points in the treatment of such a disorder. Such methods for screening for natural binding partners of a T-cadherin polypeptide are well known in the art.

One method for the screening of a candidate polypeptide interacting with a T-cadherin polypeptide comprises the following steps:
a) providing a polypeptide consisting of a T-cadherin polypeptide;
b) obtaining a candidate polypeptide;
c) bringing into contact said polypeptide with said candidate polypeptide; and
d) detecting the complexes formed between said polypeptide and said candidate polypeptide.

In the screening method defmed above, the complexes formed between the polypeptide and the candidate polypeptide can be further incubated in the presence of a polyclonal or a monoclonal antibody that specifically binds to the T-cadherin polypeptide. Alternatively, the complexes formed between the polypeptide and the candidate polypeptide are detected by performing a FACS binding assay.

In the screening method, the candidate can be the expression product of a DNA insert contained in a vector. For example, such a screening method may be performed as described in Examples 1 and 2 of the present specification.

In the screening method, the binding partners can be identified through a two-hybrid screening assay. The yeast two-hybrid system is designed to study protein-protein interactions *in vivo* (Fields and Song, 1989), and relies upon the fusion of a bait protein to the DNA binding domain of the yeast Gal4 protein. This technique is also described in US Patent Nos. 5,667,973 and 5,283,173. The general procedure of library screening by the two-hybrid assay may for example be performed as described by Fromont-Racine *et al.* (1997), the bait polypeptide consisting of a T-cadherin polypeptide. More precisely, a T-cadherin polynucleotide is fused in frame to a polynucleotide encoding the DNA binding domain of the GAL4 protein, the fused nucleotide sequence being inserted in a suitable expression vector, for example pAS2 or pM3.

In the screening method, the binding partners can also be identified through affinity chromatography. The T-cadherin polypeptide may be attached to the column using conventional techniques including chemical coupling to a suitable column matrix (*e.g*. agarose, AFFI GEL, *etc.).* In some embodiments of this method, the affinity column contains chimeric proteins in which the T-cadherin polypeptide, or a fragment thereof, is fused to glutathion S transferase (GST). A mixture of cellular proteins or pool of expressed proteins as described above is applied to the affinity column. Polypeptides interacting with the T-cadherin polypeptide attached to the column can then be isolated and analyzed, *e.g.*, on 2-D electrophoresis gel as described in Rasmunsen *et al.* (1997). Alternatively, the proteins retained on the affinity column can be purified by electrophoresis-based methods and sequenced.

In the screening method, the binding partners can also be identified through optical biosensor methods (see, *e.g.*, Edwards and Leatherbarrow, 1997). This technique permits the detection of interactions between molecules in real time, without the need of labeled molecules.

Preferably, all assays comprising the step of testing the binding of a T-cadherin polypeptide to a candidate compound, a candidate modulator or a candidate natural binding partner are performed in the presence of a divalent cation. Most preferably, such assays are performed in the presence of Ca²⁺.

The present invention is directed to the use of a soluble form of T-cadherin for the preparation of a medicament for the treatment of a a metabolic disorder. Soluble forms of T-cadherin can easily be generated as indicated above. The soluble form of T-cadherin can act as a T-cadherin agonist.

A further aspect of the present specification is directed to a method of assessing the efficiency of a modulator of a T-cadherin polypeptide for the treatment of a disorder selected from the group consisting of a metabolic disorder, a gynecologic disorder, a chronic inflammatory disorder and a liver or renal disorder, said method comprising administering said modulator to an animal model for said disorder, wherein a determination that said modulator ameliorates a representative characteristic of said disorder in said animal model indicates that said modulator is a drug for the treatment of said disorder. Said disorder is preferably a metabolic disorder. Said metabolic disorder is preferably selected from the group consisting of obesity, type II diabetes, insulin resistance, hypercholesterolemia, hyperlipidemia, dyslipidemia, syndrome X, anorexia and cachexia.

Preferably, the disorder is obesity. One example of a method that can be used for screening for drugs for the treatment of obesity and/or for assessing the efficiency of an modulator of a T-cadherin polypeptide for the treatment of obesity is a method comprising the step of administering said modulator to an animal model for obesity, wherein a determination that said modulator ameliorates a representative characteristic of obesity in said animal model indicates that said modulator is a drug for the treatment of obesity.

Animal models for obesity and assays for determining whether a compound ameliorates a representative characteristic of obesity in such animal models are known by those of skill in the art. Preferred animal model for obesity include fa/fa rats, ob/ob mice, db/db mice, leptin deficient mice and leptin-receptor deficient mice (see, *e.g.*, Pelleymounter *et al.,* 1995; Ogawa *et al.,* 1995; Hu *et al.,* 1996; Piercy *et al.,* 2000; Yamauchi *et al.,* 2001).

When studying obesity, the representative characteristic may be, *e.g.*, the Body Mass Index (BMI), the body weight, and/or the percentage of body fat. Preferably, the representative characteristic is the Body Mass Index. Methods for measuring these characteristics are well known for those of skill in the art. A determination that a modulator of a T-cadherin polypeptide reduces the body weight of an animal model for obesity can indicate that said modulator is a drug for the treatment of obesity. Preferably, a 5%, 10%, 15%, 20%, 25%, 30%, 40%, 50% or greater reduction of the body weight indicates that said modulator is a drug for the treatment of psoriasis. Most preferably, a reduction of 10% or more of the body weight indicates that said modulator is a drug for the treatment of obesity.

Preferably, the disorder is type II diabetes. One example of a method that can be used for screening for drugs for the treatment of type II diabetes and/or for assessing the efficiency of an modulator of a T-cadherin polypeptide for the treatment of type II diabetes is a method comprising the step of administering said modulator to an animal model for type II diabetes, wherein a determination that said modulator ameliorates a representative characteristic of type II diabetes in said animal model indicates that said modulator is a drug for the treatment of type II diabetes.

Animal models for type II diabetes and assays for determining whether a compound ameliorates a representative characteristic of type II diabetes in such animal models are known by those of skill in the art. Preferred animal models for type II diabetes include the C57BLKsJ diabetic mouse, the KKA(y) mouse, the Nagoya-Shibata-Yasuda (NSY) mouse, and the obese diabetic (db/db) mouse (see, *e.g.*, Castle *et al.,* 1993; Piercy *et al.,* 1998; Piercy *et al.,* 2000; Ueda *et al.,* 2000).

Determining whether the modulator ameliorates a representative characteristic of diabetes may be performed using several methods available in the art. For example, the representative characteristic may be the plasma, serum or blood levels of glucose. In one embodiment, the representative characteristic is the fasting plasma glucose (FPG) level. Alternatively, the representative characteristic is the level of postprandial glucose. The representative characteristic may also be the level of fructosamine and glycated hemoglobin (HbA1c) Both HbA1c levels and FPG levels are commonly used measure in clinical trials for type II diabetes treatments (see, *e.g.*, Holman *et al.,* 1999). Alternatively, the representative characteristic may be the level of total cholesterol, HDL cholesterol, LDL cholesterol and/or triglyceride (see, *e.g.*, Feinglos *et al.,* 1997). Methods for measuring the above representative characteristic are well known in the art.

A determination that a modulator of a T-cadherin polypeptide reduces HbA1c levels of at least 0.5%, 0.6%, 0.7%, 08%, 0.9%, 1%, 2%, 5%, 10%, 15%, 20% or more relative to placebo can indicate that said modulator is a drug for the treatment of type II diabetes. As used herein, the term "placebo" refers to an animal model to which said modulator of a T-cadherin polypeptide has not been administered.

Reference to known method steps, conventional methods steps, known methods or conventional methods is not in any way an admission that any aspect, description or embodiment of the present invention is disclosed, taught or suggested in the relevant art.

### Examples

### Example 1: Construction and expression of tagged Acrp30

The entire coding sequence for murine Acrp30 (SEQ ID NO: 3) was inserted in the pCDNA3.1 vector. The pcDNA3.1 vector was obtained from Invitrogen (Carlsbad, CA). The pCDNA3.1 vector includes the CMV promoter that regulates expression of the cloned gene.

The coding sequence of the Flag epitope (Asp-Tyr-Lys-Asp-Asp-Asp-Asp-Lys) was inserted by PCR mutagenesis between the predicted signal sequence cleavage site of Acrp30 (amino acid at position 17 of SEQ ID NO: 3) and the amino-terminal region. This construct is denoted 5'FlagAcrp30. The PCR mutagenesis was performed using primers of SEQ ID NOs: 5 to 8. Primers of SEQ ID NOs: 6 and 7 contain the sequence encoding the Flag epitope and Acrp30 sequences, and were used in overlap PCR to introduce the tag after the signal sequence. SEQ ID Nos. 5 and 8 contain Acrp30 sequences at the 5' and 3' ends of the gene respectively, and also contain an EcoRI site used for subcloning into pCDNA3.1.

A second construct, denoted 3'Flag-Acrp30, was also constructed by inserting by PCR mutagenesis the coding sequence for the Flag epitope immediately before the stop codon of the Acrp30 cDNA to generate carboxyl-terminal tagged protein. The PCR mutagenesis was performed using primers of SEQ ID NO: 9, which contains Acrp30-5' sequences preceded by an EcoRI site, and of SEQ ID NO: 10, which contains the Acrp30-3' sequences lacking the stop codon, the stop codon being replaced with a sequence encoding the Flag epitope immediately followed by a stop codon and an EcoRI site.

These two Flag-Acrp30 constructs were sequenced to confirm that no PCR introduced errors had occurred.

To generate recombinant Flag-Acrp30 proteins, HEK cells were transiently transfected with these constructs. After transferring the cells to serum-free media, the supernatants were collected. The concentration of protein was determined by comparison with known amounts of purified bacterial-expressed Flag-tagged Acrp30 fusion protein by Western blot assay. The supernatants contained approximately 5 µg/ml of secreted tagged Acrp30.

These supernatants were further purified by ammonium sulfate precipitation followed by FPLC-anion-exchange chromatography (HiQ, Bio-Rad) of the resuspended proteins. After anion chromatography, native gel-electrophoresis and immunoblotting for the Flag epitope showed resolution of the expressed protein into two species: the first contained predominantly trimeric S'Flag-Acrp30, while the second contained hexameric and higher molecular weight 5'Flag-Acrp30. The purity of these preparations was judged to be approximately 50 percent by SDS-PAGE and Coomassie staining.

### Example 2: Binding of Flag-Acrp30 polypeptides to C2C12 myocytes, CHO cells and Ba/F3 cells

The ability of the Flag-Acrp30 polypeptides comprised in the above supernatants to bind to undifferentiated C2C12 myocytes, to CHO cells and to Ba/F3 cells was tested by Fluorescence-activated cell-sorter (FACS) binding assays.

Adherent cells (C2C12 and CHO) or suspension cells (Ba/F3) were transferred to serum-free media for two hours and then incubated at 4°C for thirty minutes to block endocytosis. The cells were incubated in 1% BSA in PBS containing 0.9mM CaCl₂ and 0.5mM MgCl₂ (PBS++) at 4°C for thirty minutes to block non-specific binding sites. All subsequent steps were done at 4°C. The cells were incubated in a solution of 1:1 conditioned media from HEK cells and blocking solution for two hours, washed twice in PBS++, and incubated for one hour with a monoclonal antibody recognizing the Flag epitope conjugated to the fluorescent dye APC (Phyco Link). The antibodies recognizing the Flag epitope were purchased from Sigma (St. Louis, MO). After washing twice in PBS++, adherent cells were scraped and resuspended in PBS containing 10% FBS and 0.5 µg/ml propidium iodide (PI), a marker for cell permeability, and analyzed by FACS. The median fluorescence of live, PI-negative cells, was determined.

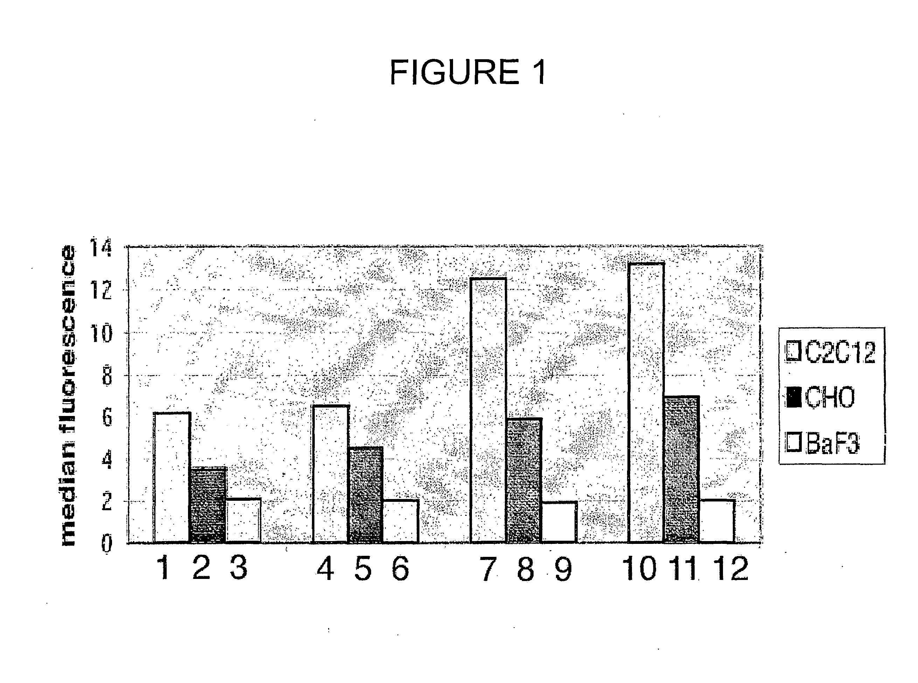

Figure 1 shows the result of the FACS binding assay. Median cell fluorescence for each cell type is shown. Lanes 1-3 correspond to a binding assay performed with buffer controls. Lanes 4-6 correspond to a binding assay performed with conditioned media from HEK cells transfected with the pCDNA3.1 vector alone, lacking any cDNA insert (control cells). Lanes 7-9 correspond to a binding assay performed with conditioned media from 5'Flag-Acrp30 transfected HEK cells. Lanes 10-12 correspond to a binding assay performed with conditioned media from 3'Flag-Acrp30 transfected HEK cells. Two independent experiments were performed. Conditioned media refers to unpurified supernantants generated as described in Example 1.

As shown in. Figure 1, Ba/F3 cells did not bind substantially to either 5'FlagAcrp30 (lane 9) or 3'FlagAcrp30 (lane12). The signal obtained was similar to that obtained with either control transfected supernatants (lane 6), or when only blocking solution (lane 3) was added. CHO cells yielded a slightly greater signal when incubated in conditioned media containing either 5'Flag-tagged Acrp30 (lane 8) or 3'Flag-tagged Acrp30 (lane 11) containing media, compared to the control of only blocking protein (lane 3) or control transfected supernatant (lane 5). C2C12 cells, however, demonstrated a two-fold increase in signal when cells were incubated in conditioned media containing either 5'Flag-Acrp30 (lane7) or 3'Flag-Acrp30 (lane 10), compared to blocking solution (lane 1) or control transfected supernatant (lane 4).

These results indicate that C2C12 cells, but not Ba/F3 cells, specifically bind Acrp30.

### Example 3: Cloning of a novel Acrp30 receptor

### 3.1. C2C12 cDNA library construction

The C2C12 cell line was chosen for construction of a cDNA expression library as it binds to unpurified Flag-Acrp30 polypeptides and as it previously has been shown to increase fatty acid oxidation in response to Acrp30 (Fruebis *et al.,* 2001).

An undifferentiated C2C12 cDNA expression library was made in a bicistronic retroviral vector pBI-GFP. This vector contains the coding sequence of green fluorescent protein (GFP) under the control of an internal ribosome entry site (IRES) (Bogan *et al.,* 2001). Expression of the cloned gene or cDNA insert is proportional to the expression of GFP in individual cells.

The quality of mRNA was verified by Northern blot analysis for the p-actin gene. After ligation of the reverse transcribed cDNA into the retroviral vector, characterization of the unamplified library revealed approximately 0.5-1x10⁷ independent transformants. By restriction digestion of plasmids prepared from individual clones of the primary library, 95 percent contained inserts with an average size of 1.5 kb. Infectious cDNA viral particles were produced by transfection of the plasmid into a packaging cell line (Naviaux *et al.,* 1996). The resulting virus-containing supernatant was used to infect approximately 5 percent of a population of 2x10⁸ naïve Ba/F3 cells.

### 3.2. Preparation of magnetic beads

Magnetic beads containing tosyl-reactive groups (M280 Dynalbeads, Dynal) were incubated with anti-Flag antibody (M2, Sigma) to couple the antibody to the beads. After coupling, the beads were blocked in a solution of PBS++ containing 1% BSA for one hour, added to 40 ml of conditioned media from HEK cells transfected with 5'Flag-Acrp30, and incubated overnight at 4°C. Control beads were incubated with the supernatants of HEK cells transfected with the empty vector pCDNA3.1. After binding to the supernatants, the beads were washed twice in PBS++ containing 0.1% BSA and stored under sterile conditions in the same buffer.

### 3.3. Magnetic bead panning

The Ba/F3 cells infected by the C2C12 cDNA expression library were expanded for two days before being subjected to binding on the magnetic beads. The cells were prepared and blocked as described in Example 2, except that 0.1 mg/ml mouse IgG (Sigma) was included in the blocking step. All subsequent steps were performed at 4°C to prevent internalization of the beads.

The cells were pre-cleared to remove non-specifically bound cells by incubating 30 ml of cells (2.4x10⁷/ ml) with 30 µl of control magnetic beads for one hour. Bound cells were separated by use of a magnet, and non-adherent cells were again subjected to two additional rounds of binding to control beads. Non-adherent cells were incubated with 75 µl of 5'Flag-Acrp30 beads for one hour, after which adherent cells were separated with a magnet and washed three times (five minutes in 10 ml of PBS++ with 0.1% BSA.) The bound cells were expanded in culture; in subsequent rounds of binding, pre-clearing was performed twice with 15 µl of control beads before adding 30 µl of 5'Flag-Acrp30 beads. After each round of binding and expansion, aliquots of cells were analyzed by FACS for GFP expression to follow enrichment of cells containing an integrated retrovirus.

Since the GFP expression from the IRES of the integrated retroviral vector is proportional to the expression of the cloned cDNA insert in individual cells (Liu *et al.,* 2000), the enrichment in GFP expression in a cell population after binding to 5'Flag-Acrp30 is linked to enrichment of a cloned cDNA conferring binding.

### 3.4. FACS analysis of enriched cell pools

After one round of binding to the beads, 2.4 % of cells purified on control beads, and 1.6 % of cells purified on 5'Flag-Acrp30 beads were GFP-positive relative to uninfected cells.

After the second round of binding, 1.7 % of cells purified on controls beads and 7.8 % of cells purified on 5'Flag-Acrp30 beads were GFP-positive.

By the third sort, cells binding to control beads had not enriched compared to the first sort, as 2.3 % were GFP-positive. On the other hand, 73 % of cells binding to the 5'Flag-Acrp30 beads were GFP-positive, indicating that the population had enriched for cells containing an integrated retroviral cDNA clone.

As cells bound to the control bead did not enrich for GFP during the third sort, epigenetic effects due to enrichment of cells bound to the 5'Flag-Acrp30 beads is excluded. Thus the enriched cell population likely contains a cDNA encoding a receptor for Acrp30.

### 3.5. Amplification of enriched cDNA insert

Genomic DNA was prepared from the different cell pools obtained after the third sort and subjected to PCR amplification using retroviral specific primers flanking the cDNA cloning site of the retroviral vector pBI-GFP. These different cell pools corresponded to naïve Ba/F3 cells or to Ba/F3 cells infected with the cDNA library and bound to magnetic beads that had been incubated with the supernatant of (i) HEK cells transfected with the pcDNA3.1 vector alone; (ii) 5'FlagAcrp30 expressing HEK cells; or (ii) 5'FlagAcrp30 expressing HEK cells. In cells incubated with the 5'Flag-Acrp30 containing beads, but not in control cells or in naïve Ba/F3 cells, a single specific band, of 2.5 kb, was seen.

This band was subcloned into the vector pCRII-Topo and sequenced the ends using vector specific primers. The sequence was identical to the murine T-cadherin (GenBank accession number BC021628). In order to obtain the entire insert sequence, primers of SEQ ID NOs: 11 to 18 were designed from the know sequence of T-cadherin to enable sequencing of the entire insert. This confirmed the identity of the enriched clone as full-length T-cadherin.

Accordingly, T-cadherin is a potential Acrp30 receptor. The fact that T-cadherin is an Acrp30 receptor was confirmed as further detailed below.

### Example 4: Binding of Acrp30 to T-cadherin

### 4.1. Over-expression of T-cadherin in cells

Full-length murine T-cadherin cDNA was obtained as an expressed sequence tag (IMAGE clone ID 3987627) and cloned into the pCDNA3.1 vector to generate pCDNA-Tcad construct. Three cell lines over-expressing T-cadherin were generated.

CHO cells were transiently transfected with pCDNA-Tcad. These transiently transfected CHO cells demonstrated increased binding to 5'Flag-Acrp30 compared to control tissue culture supernatants using the FACS binding assay previously described.

The entire T-cadherin coding sequence was cloned into the retroviral pBI-GFP vector to generate the pBI-GFP-Tcad construct. Both naïve Ba/F3 and CHO-ER cells were transfected with pBI-GFP-Tcad. The CHO-ER cell line stably expresses the retroviral vector receptor and can be infected by ecotropic viruses (gift of Dr. M. Krieger, MIT).

### 4.2. Analysis of Acrp30 binding to T-cadherin by FACS

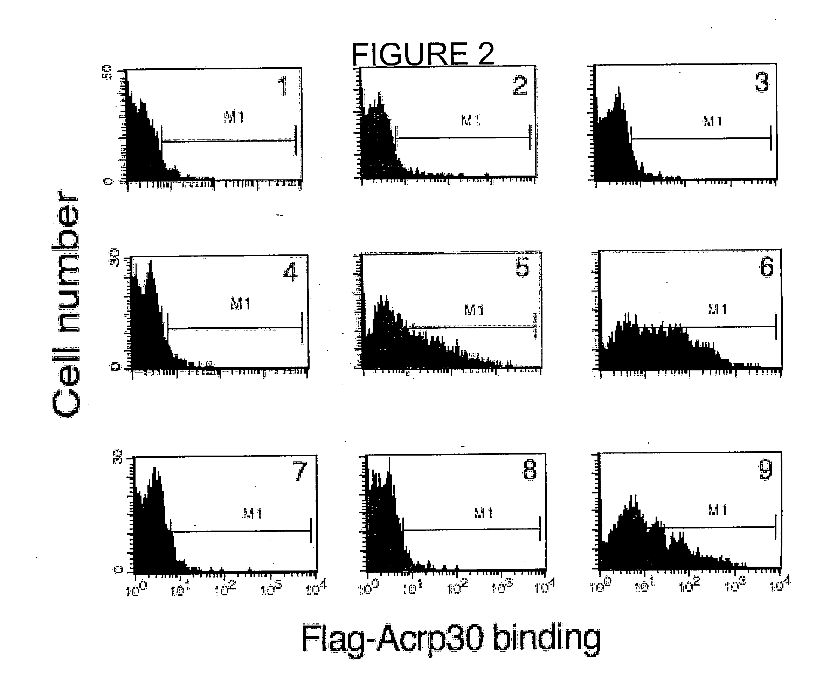

The above cell lines were subsequently used for FACS binding assays, as shown on Figure 2. Panels 1 to 3 correspond to FACS binding assay of control Ba/F3 cells. Panels 4 to 9 correspond to FACS binding assay of retroviral infected T-cadherin (pBI-GFP-Tcad) expressing Ba/F3 cells. Binding was studied for:
- 0 nM (panels 1 and 4), 6 nM (panels 2 and 5, 7-9), or 60 nM (panels 3 and 6) of 5'Flag-Acrp30 hexamer;
- 60 nM Acrp30 hexamer (panel 7);
- 10 mM EDTA (panel 8); and
- 10 µg/ml C1q (panel 9).

Control, uninfected Ba/F3 cells exhibited low binding to 6 nM (panel 2) or 60 nM (panel 3) 5'Flag-Acrp30 hexamer, while Ba/F3 cells expressing T-cadherin demonstrated increasing binding with 6 nM (panel 5, and panels 7-9) or 60 nM (panel 6) 5'Flag-Acrp30 hexamer (concentration expressed as trimer-equivalents). Background binding in the absence of ligand was low (panel 4). Including 60 nM of eukaryotic produced, untagged Acrp30 hexamer inhibited binding of 6 nM 5'FlagAcrp30, (panel 7), indicating specific binding between Acrp30 and T-cadherin. To examine the divalent cation requirements for binding, 10 mM EDTA were added to the binding reaction. This completely blocked binding (panel 8), indicating divalent cations are required for binding. C1q, a molecule that shares homology to Acrp30, did not affect binding at a 20-fold excess (by weight) when co-incubated with 6 nM 5'Flag-Acrp30 hexamer (panel 9), indicating that C1q likely does not bind to the same receptor as Acrp30.

In parallel experiments, no significant binding of bacterial-expressed gAcrp30 neither to T-cadherin expressing cell lines, nor to a preparation of trimeric mammalian-cell-produced 5'Flag-Acrp30 was seen.

This experiment confirms that T-cadherin is not binding to the Flag epitope, thus ruling out a trivial explanation for the binding to 5'Flag-Acrp30. Additionally, this result suggests that T-cadherin is a specific receptor for full-length hexameric and HMW species of Acrp30, but does not bind the globular or trimeric species of Acrp30.

### 4.3. Analysis of Acrp30 binding to T-cadherin by ELISA

ELISA assays were further performed to show direct binding of Acrp30 to cells expressing T-cadherin.

CHO-ER cell lines infected with the bicistronic retroviral construct pBI-GFP-Tcad (referred to as CHO-T-cadherin) and control cells infected with pBI-GFP (referred to as CHO-GFP) were each grown in 96 well plates. One day after plating (1.5 x10⁴/well), the cells were incubated in serum-free media for one hour, placed at 4°C for thirty minutes, and blocked in PBS++ containing 4 % dried milk for thirty minutes. Bacterial expressed Flag-tagged globular-Acrp30, bacterial expressed Flag-tagged full-length, or purified trimeric or hexameric and HMW 5'Flag-Acrp30 produced in HEK cells were incubated with the cells for one hour in blocking buffer at the indicated concentrations. The HEK expressed polypeptides were produced and purified by ammonium sulfate precipitation and anion-exchange chromatography. Pools containing the trimeric or a combination of hexamer and HMW 5'Flag-Acrp30 were identified by native gel electrophoresis and immunoblotting with a Flag antibody. The purity of these preparations was judged to be approximately 50 % by Coomassie PAGE analysis. Protein concentration was determined by BCA assay (Pierce). The concentration of purified bacterial expressed proteins was determined by the absorbance at 280 nm and the calculated extinction coefficient derived from the primary amino acid sequence (Protean; DNAStar). After washing twice in PBS ++, a monoclonal antibody to the Flag epitope (M2; 4 µg/ml) was added for one hour; washing was repeated, followed by an incubation in secondary antibody conjugated to horseradish peroxidase (donkey anti-mouse; Jackson), and developed with colorimetric TMB substrate (Pierce). The absorbance at 450 nm was measured with a plate reader.

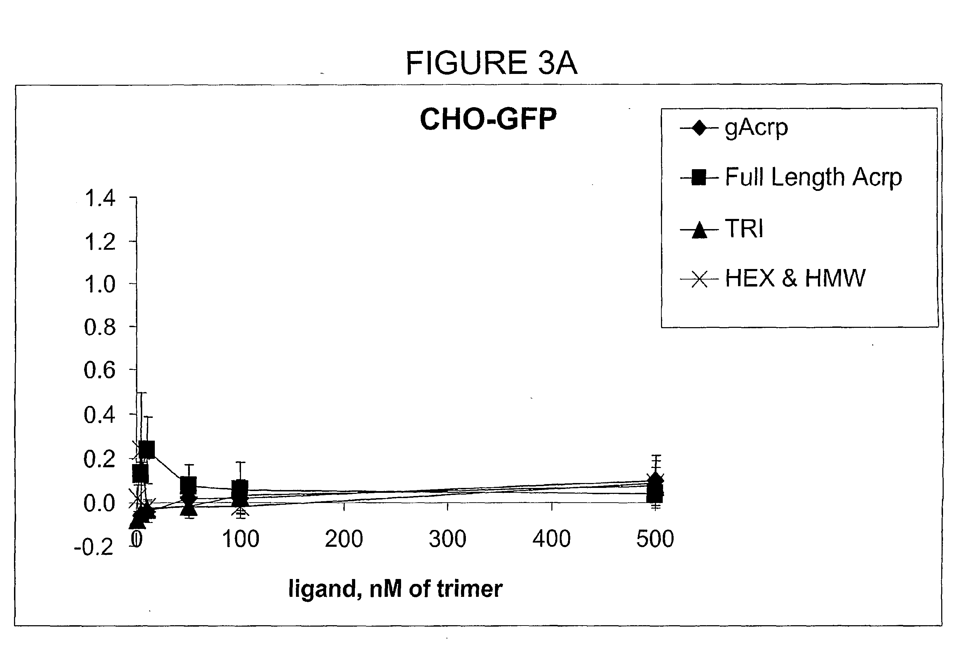

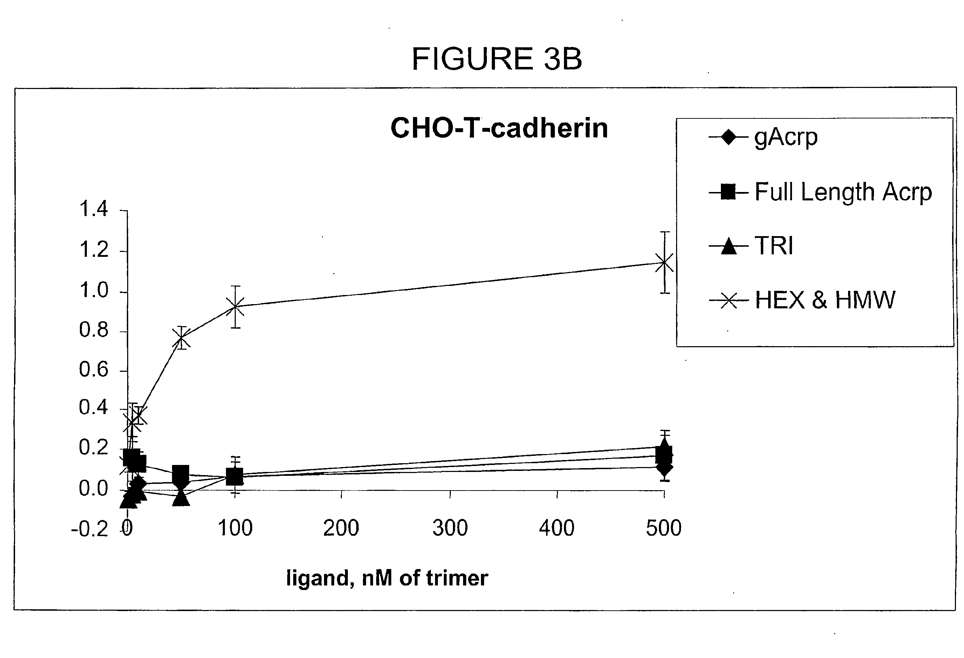

The results are shown in Figures 3A and 3B. Figure 3A shows the ELISA analysis of binding of several preparations of Acrp30 to CHO-GFP control. Figure 3B shows the CHO-T-cadherin expressing cell lines. gAcrp refers to bacterial-expressed 3'Flag-gAcrp30. Full Length Acrp refers to bacterial-expressed 3'Flag-Acrp30. TRI refers to HEK produced trimeric 5'FlagAcrp30. HEX & HMW refers to HEK produced hexameric and high-molecular weight 5'FlagAcrp30. Four independent experiments were performed.

As seen in Figure 3A, control cells did not bind any of the tested proteins. As seen in Figure 3B, CHO cells expressing T-cadherin demonstrated binding only to hexameric and HMW, but not trimeric, oligomers of 5'FlagAcrp30. The estimated half-maximal binding concentration is of 25-50 nM (concentration expressed as trimer equivalents). There was no binding of either globular or full-length bacterial produced protein to CHO cells expressing T-cadherin, implying that recognition of Acrp30 by T-cadherin may require post-translational modifications to Acrp30 and does likely not involve the globular domain.
Accordingly, T-cadherin can bind to the hexameric and HMW species of Acrp30, but not to the globular or trimeric species.

### Example 5: Effect of T-cadherin on NF-κB-mediated transcription

To determine whether cells over-expressing T-cadherin have an altered NF-κB response to Acrp30 stimulation, NF-κB-mediated transcription in cells transiently transfected with either the control pCDNA3.1 plasmid or the pCDNA-T-cadherin plasmid (pCDNA-Tcad) was examined as described in Tsao *et al.* (2002).

To assay for NF-κB activity, pCDNA-Tcad was co-transfected with a plasmid containing the luciferase coding sequence under the control of an E-selectin promoter encoding an NF-κB response element. The E-selectin luciferase construct was generated by inserting the E-selected promoter into the pGL2-Basic vector containing the luciferase gene (Promega) as described by Schindler and Baichwal (1994). A third plasmid expressing β-galactosidase under the control of the CMV promoter was co-transfected as well and was used to normalize levels of expression. The plasmids were transfected into cells in 24 well plates. The next day, the cells were incubated for six hours in media containing the indicated compounds. The luciferase and β-galactosidase enzymatic activity of the cell lysates were determined with a luminometer based assay (Promega). The luciferase signal was normalized to β-galactosidase activity and plotted as the fold-stimulation relative to unstimulated cells transfected with the identical constructs.

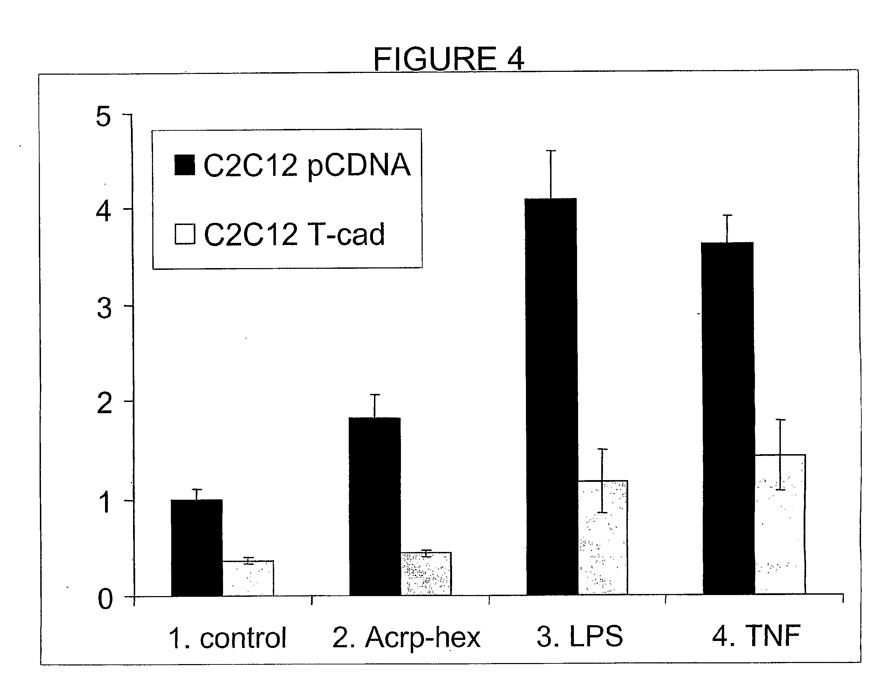

Figure 4 shows the fold-stimulation of NF-κB-mediated transcription in undifferentiated C2C12 myocytes. Luciferase activity was measured. Column 1 shows the fold-stimulation following 6 h treatment with media alone. Column 2 shows the fold-stimulation with addition of 2 ug/ml hexameric Acrp30. Column 3 shows the fold-stimulation with addition of 300 ng/ml LPS. Column 4 shows the fold-stimulation with addition of 50 ng/ml TNF-α. Three independent experiments were performed.

Column 1 shows that T-cadherin expression suppresses basal NF-κB-mediated transcription to 37 % of the NF-κB-mediated transcription in control cells. The Acrp30 hexamer (column 2) at a concentration of 2 ug/ml stimulates NF-κB-mediated transcription 1.8-fold. In T-cadherin expressing cells, this stimulation is completely suppressed, since the NF-κB-mediated transcription has the same level as in untreated T-cadherin expressing cells. Columns 3 and 4 show C2C12 cells treated with either 300 ng/ml LPS (column 3) or 50 ng/ml TNF-α (column 4). In both cases, there is a similar four-fold stimulation in control transfected cells, and in both cases, the stimulation was reduced by T-cadherin expression. However, T-cadherin only reduced the stimulation to the base level of control transfected cells. There was not further suppression as was the case in cells treated with hexameric Acrp30. β-galactosidase expression was unchanged between pCDNA and T-cadherin transfected cell lines, indicating that over-expression of T-cadherin did not lead to non-specific transcriptional suppression.

In conclusion, over-expression of T-cadherin suppresses NF-κB-mediated transcription initiated by several different stimuli, including NF-κB-mediated transcription initiated by Acrp30.

### References

1. Arita, Kihara et al. (1999) "Paradoxical decrease of an adipose-specific protein, adiponectin, in obesity" Biochem Biophys Res Commun 257(1): 79-83
2. Berg, Combs et al. (2001) "The adipocyte-secreted protein Acrp30 enhances hepatic insulin action" Nat Med 7(8): 947-53
3. Bogan, McKee et. al. (2001) "Insulin-responsive compartments containing GLUT4 in 3T3-L1 and CHO cells: regulation by amino acid concentrations" Mol Cell Biol 21(14): 4785-806
4. Castle, Colca et al. (1993) "Lipoprotein profile characterization of the KKA(y) mouse, a rodent model of type II diabetes, before and after treatment with the insulin-sensitizing agent pioglitazone" Arterioscler Thromb 13(2):302-9
5. Chebath J, Fischer et al. (1997) "Interleukin-6 receptor-interleukin-6 fusion proteins with enhanced interleukin-6 type pleiotropic activities" Eur Cytolcine Netw. 8(4):359-65.
6. Conacci-Sorrell, Zhurinsky et al. (2002) "The cadherin-catenin adhesion system in signaling and cancer" J Clin Invest 109(8): 987-91
7. Doyle, Goings et al. (1998) "T-cadherin is a major glycophosphoinositol-anchored protein associated with noncaveolar detergent-insoluble domains of the cardiac sarcolemma" J Biol Chem 273(12): 6937-43
8. Edwards and Leatherbarrow "determination of association rate constants by an optical biosensor using initial rate analysis" Anal Biochem, 1997 Mar 1;246(1):1-6
9. Elbashir, Harborth et al. (2001) Duplexes of 21-nucleotide RNAs mediate RNA interference in cultured mammalian cells Nature, 2001 May 24;411(6836):494-8
10. Ellington and Szostak (1990) "In vitro selection of RNA molecules that bind specific ligands" Nature 346(6287):818-22
11. Elliott and Pestronk (1994) "Progression of multifocal motor neuropathy during apparently successful treatment with human immunoglobulin" Neurology. 44(5):967-8.
12. Febbraio and Pedersen (2002) "Muscle-derived interleukin-6: mechanisms for activation and possible biological roles" FASEB J 16(11):1335-47
13. Feinglos, Thacker et al. (1997) "Modification of postprandial hyperglycemia with insulin lispro improves glucose control in patients with type 2 diabetes" Diabetes Care, 20(10):1539-42
14. Fields and Song (1989) "A novel genetic system to detect protein-protein interactions" Nature 340(6230):245-6 Nature 340:245-6
15. Fromont-Racine, Rain et al. (1997) Toward a functional analysis of the yeast genome through exhaustive two-hybrid screens Nat Genet 16(3):277-82
16. Fruebis, Tsao et al. (2001) "Proteolytic cleavage product of 30-kDa adipocyte complement-related protein increases fatty acid oxidation in muscle and causes weight loss in mice" Proc Natl Acad Sci U S A 98(4):, 2005-10
17. Grantham R (1974) "Amino acid difference formula to help explain protein evolution" Science 185(4154):862-4
18. Hara, Boutin et al. (2002) "Genetic variation in the gene encoding adiponectin is associated with an increased risk of type 2 diabetes in the Japanese population" Diabetes 51(2): 536-40
19. Holman, Cull et al. (1999) "A randomized double-blind trial of acarbose in type 2 diabetes shows improved glycemic control over 3 years" Diabetes Care 22(6):960-4
20. Hu, Liang et al. (1996) "AdipoQ is a novel adipose-specific gene dysregulated in obesity" J Biol Chem 271(18): 10697-10703
21. Huang, Wu et al. (2003) "T-cadherin-mediated cell growth regulation involves G2 phase arrest and requires p21(CIP1/WAFl) expression" Mol Cell Biol 23(2): 566-78
22. Ivanov, Philippova et al. (2001) "Expression of cell adhesion molecule T-cadherin in the human vasculature" Histochem Cell Biol 115(3): 231-42
23. Knight, Trinh et al. (1993) "Construction and initial characterization of a mouse-human chimeric anti-TNF antibody" Mol Immunol. 30(16): 443-53.
24. Kollet O, Aviram et al. (1999) "The soluble interleulcin-6 (IL-6) receptor/IL-6 fusion protein enhances in vitro maintenance and proliferation of human CD34(+)CD38(-/low) cells capable of repopulating severe combined immunodeficiency mice" Blood. 94(3):923-31.
25. Kondo, Shimomura et al. (2002) "Association of adiponectin mutation with type 2 diabetes: a candidate gene for the insulin resistance syndrome" Diabetes 51(7): 2325-8
26. Kosmidou, Vassilakopoulos et al. (2002) "Production of interleukin-6 by skeletal myotubes: role of reactive oxygen species " Am J Respir Cell Mol Biol 26(5):587-93
27. Lindsay, Funahashi et al. (2002) "Adiponectin and development of type 2 diabetes in the Pima Indian population" Lancet 360(9326): 57-8
28. Liu, Constantinescu et al. (2000) "Generation of mammalian cells stably expressing multiple genes at predetermined levels" Anal Biochem 280(1):, 20-8
29. Maeda, Okubo et al. (1996) "cDNA cloning and expression of a novel adipose specific collagen-like factor, apM1 (AdiPose Most abundant Gene transcript 1)" Biochem Biophys Res Commun 221(2): 286-9
30. Maeda, Shimomura et al. (2002) "Diet-induced insulin resistance in mice lacking adiponectin/ACRP30" Nat Med 8(7): 731-7
31. Naviaux, Costanzi et al. (1996) "The pCL vector system: rapid production of helper-free, high-titer, recombinant retroviruses" J Virol 70(8): 5701-5
32. Niermann, Kern et al. (2000) "The glycosyl phosphatidylinositol anchor of human T-cadherin binds lipoproteins" Biochem Biophys Res Commun 276(3): 1240-7
33. Ogawa, Masuzaki et al. (1995) "Molecular cloning of rat obese cDNA and augmented gene expression in genetically obese Zucker fatty (fa/fa) rats" J Clin Invest 96(3):1647-52
34. Okamoto, Arita et al. (2000) "An adipocyte-derived plasma protein, adiponectin, adheres to injured vascular walls" Horm Metab Res 32(2): 47-50
35. Panidis, Kourtis et al. (2003) "Serum adiponectin levels in women with polycystic ovary syndrome" Hum Reprod. 18(9):1790-6.
36. Pelleymounter, Cullen et al. (1995) "Effects of the obese gene product on body weight regulation in ob/ob mice Science 269(5223):540-3"
37. Petridou, Mantzoros et al. (2003) "Plasma adiponectin concentrations in relation to endometrial cancer: a case-control study in Greece" J Clin Endocrinol Metab. 88(3):993-7.
38. Piercy, Toseland et al. (1998) "Potential benefit of inhibitors of advanced glycation end products in the progression of type II diabetes: a study with aminoguanidine in C57/BLKsJ diabetic mice" Metabolism 47(12):1477-80
39. Piercy, Toseland et al. (2000) « Acceleration of the development of diabetes in obese diabetic (db/db) mice by nicotinamide: a comparison with its antidiabetic effects in non-obese diabetic mice" Metabolism, 2000 Dec;49(12):1548-54
40. Ramsay, Jamieson et al. (2003) "Paradoxical elevation in adiponectin concentrations in women with preeclampsia." Hypertension. 42(5):891-4.
41. Rasmussen, Ji et al. (1997) "Two-dimensional electrophoretic analysis of human breast carcinoma proteins: mapping of proteins that bind to the SH3 domain of mixed lineage kinase MLK2" Electrophoresis, 1997 Mar-Apr;18(3-4):585-98
42. Roth, Mobarhan et al. (2002) "Syndrome X: where are we and where do we go?" Nutr Rev 60:335-7 Review
43. Sambrook, Fritsch et al. (1989) "Molecular Cloning: A Laboratory Manual" Cold Spring Harbor Laboratory Press, Cold Spring Harbor, NY
44. Scherer, Williams et al. (1995) "A novel serum protein similar to C1q, produced exclusively in adipocytes" J Biol Chem 270(45): 26746-9
45. Schindler and Baichwal (1994) Three NF-kappa B binding sites in the human E-selectin gene required for maximal tumor necrosis factor alpha-induced expression. Mol Cell Biol. 14(9):5820-31.
46. Takeuchi, Misaki et al. (2000) "Expression of T-cadherin (CDH13, H-Cadherin) in human brain and its characteristics as a negative growth regulator of epidermal growth factor in neuroblastoma cells" J Neurochem 74(4): 1489-97
47. Tanihara, Sano et al. (1994) "Cloning of five human cadherins clarifies characteristic features of cadherin extracellular domain and provides further evidence for two structurally different types of cadherin" Cell Adhes Commun 2(1): 15-26
48. Tkachuk, Bochkov et al. (1998) "Identification of an atypical lipoprotein-binding protein from human aortic smooth muscle as T-cadherin" FEBS Lett 421(3):, 208-12
49. Tsao, Murrey et al. (2002) "Oligomerization state-dependent activation of NF-kappa B signaling pathway by adipocyte complement-related protein of 30 kDa (Acrp30)" J Biol Chem 277(33): 29359-62
50. Tsao, Tomas et al. (2003) "Role of Disulfide Bonds in Acrp30/Adiponectin Structure and Signaling Specificity: Different Oligomers Activate Different Signal Transduction Pathways" J Biol Chem In the press
51. Ueda, Ikegami et al. (2000) "Age-dependent changes in phenotypes and candidate gene analysis in a polygenic animal model of Type II diabetes mellitus; NSY mouse" Diabetologia 43(7):932-8
52. Xu, Wang et al. (2003) "The fat-derived hormone adiponectin alleviates alcoholic and nonalcoholic fatty liver diseases in mice" J Clin Invest. 2003 Ju1;112(1):91-100.
53. Yamauchi, Kamon et al. (2003) "Cloning of adiponectin receptors that mediate antidiabetic metabolic effects" Nature 423(6941): 762-9
54. Yamauchi, Kamon et al. (2001) "The fat-derived hormone adiponectin reverses insulin resistance associated with both lipoatrophy and obesity" Nat Med 7(8): 941-6
55. Yokota, Oritani et al. (2000) "Adiponectin, a new member of the family of soluble defense collagens, negatively regulates the growth of myelomonocytic progenitors and the functions of macrophages" Blood. 96(5):1723-32
56. Zoccali, Mallamaci et al. (2003) "Adiponectin is markedly increased in patients with nephrotic syndrome and is related to metabolic risk factors" Kidney Int Suppl. 84:598-102.

## Claims

1. Soluble form of T-cadherin for treatment of a metabolic disorder, wherein said soluble form is selected from the group consisting of:
a) a polypeptide consisting of amino acids 23 to 692 of SEQ ID NO: 1;
b) a polypeptide consisting of a fragment of at least 250, 300, 350, 400, 450, 500, 550, 600 or 650 amino acids of (a);
c) a mutein of (a) or (b), wherein the amino acid sequence has at least 90%, 95%, 96%, 97%, 98% or 99% identity to at least one of the sequences in (a) or (b);
d) a mutein of (a) or (b) which is encoded by a nucleic acid which hybridizes to the complement of a DNA sequence encoding (a) or (b) under highly stringent conditions; and
e) a mutein of (a) or (b) wherein any changes in the amino acid sequence are conservative amino acid substitutions of the amino acid sequences in (a) or (b).

2. The use of claim 1, wherein said disorder is a metabolic disorder selected from the group consisting of obesity, type II diabetes, insulin resistance, hypercholesterolemia, hyperlipidemia, dyslipidemia, syndrome X, anorexia and cachexia.

## Patentansprüche

1. Lösliche Form von T-Cadherin zur Behandlung von metabolische Störungen, bei welcher die genannte lösliche Form ausgewählt ist aus der Gruppe bestehend aus:
a) einem Polypeptid bestehend aus den Aminosäuren 23 bis 692 der SEQ.ID. NO. 1;
b) einem Polypeptid bestehend aus einem Fragment von wenigstens 250, 300, 350, 400,450, 500, 550, 600 oder 650 Aminosäuren von a);
c) ein Mutein von a) oder b), bei welchem die Aminosäuresequenz wenigstens 90%, 95%, 96%, 97%, 98% oder 99% Identität von wenigstens einer der Sequenzen a) oder b) aufweist;
d) ein Mutein von a) oder b), welches durch eine Nucleinsäure kodiert ist, die an ein Komplement einer DNA-Sequenz hybridisiert, die a) oder b) unter hoch stringenten Bedingungen codiert; und
e) ein Mutein von a) oder b), bei welchem alle Änderungen in der Aminosäuresequenz konservative Aminosäuresubstitutionen der Aminosäuresequenzen a) oder b) sind.

2. Verwendung nach Anspruch 1, wobei die genannte metabolische Störung ausgewählt ist aus der Gruppe bestehend aus Obesitas, Typ II Diabetes, Insulinresistenz, Hypercholeslerolämie, Hyperlipidämie, Dyslipidämie, Syndrom X, Anorexie und Kachexie.

## Revendications

1. Forme soluble de T-cadhérine destinée au traitement d'un trouble métabolique, ladite forme soluble étant sélectionnée dans le groupe constitué par :
a) un polypeptide constitué par les acides aminés 23 à 692 de SEQ ID NO : 1 ;
b) un polypeptide constitué par un fragment d'au moins 250, 300, 350, 400, 450, 500, 550, 600 ou 650 acides aminés de (a) ;
c) une mutéine selon (a) ou (b), dans laquelle la séquence d'acides aminés présente une identité d' au moins 90 %, 95 %, 96 %, 97 %, 98 % ou 99 % avec au moins une des séquences dans (a) ou (b) ;
d) une mutéine selon (a) ou (b) qui est codée par un acide nucléique qui s'hybride au complément d'une séquence d'ADN codant pour (a) ou (b) dans des conditions de stringence élevée ; et
e) une mutéine de (a) ou (b) dans laquelle toutes les modifications dans la séquence d'acides aminés sont des substitutions d'acides aminés conservatives des séquences d'acides aminés dans (a) ou (b).

2. Utilisation selon la revendication 1, dans laquelle ledit trouble est un trouble métabolique choisi dans le groupe constitué par l'obésité, le diabète de type II, l'insulinorésistance, l'hypercholestérolémie, l'hyperlipidémie, la dyslipidémie, le syndrome X, l'anorexie et la cachexie.


### SEQUENCE LISTING

<110> Whitehead Institute for Biomedical Research
   Hug, Christopher
   Lodish, Harvey F.
<120> USE OF T-CADHERIN AS A TARGET
<130> SER-100X
<150> US 60/526,956
   <151> 2003-12-03
<160> 18
<170> PatentIn version 3.1
<210> 1
   <211> 713
   <212> PRT
   <213> Homo sapiens
<220>
   <221> SIGNAL
   <222> (1)..(22)
   <223>
<220>
   <221> LIPID
   <222> (693)..(693)
   <223> GPI-ANCHOR
<220>
   <221> PROPEP
   <222> (23) .. (139)
   <223>
<220>
   <221> PROPEP
   <222> (694)..(713)
   <223>
<400> 1
   Met Gln Pro Arg Thr Pro Leu Val Leu Cys Val Leu Leu Ser Gln Val
   1               5                   10                  15
   Leu Leu Leu Thr Ser Ala Glu Asp Leu Asp Cys Thr Pro Gly Phe Gln
               20                  25                  30
   Gln Lys Val Phe His Ile Asn Gln Pro Ala Glu Phe Ile Glu Asp Gln
           35                  40                  45
   Ser Ile Leu Asn Leu Thr Phe Ser Asp Cys Lys Gly Asn Asp Lys Leu
       50                  55                  60
   Arg Tyr Glu Val Ser Ser Pro Tyr Phe Lys Val Asn Ser Asp Gly Gly
   65                  70                  75                  80
   Leu Val Ala Leu Arg Asn Ile Thr Ala Val Gly Lys Thr Leu Phe Val
                   85                  90                  95
   His Ala Arg Thr Pro His Ala Glu Asp Met Ala Glu Leu Val Ile Val
               100                 105                 110
   Gly Gly Lys Asp Ile Gln Gly Ser Leu Gln Asp Ile Phe Lys Phe Ala
           115                 120                 125
   Arg Thr Ser Pro Val Pro Arg Gln Lys Arg Ser Ile Val Val Ser Pro
       130                 135                 140
   Ile Leu Ile Pro Glu Asn Gln Arg Gln Pro Phe Pro Arg Asp Val Gly
   145                 150                 155                 160
   Lys Val Val Asp Ser Asp Arg Pro Glu Arg Ser Lys Phe Arg Leu Thr
                   165                 170                 175
   Gly Lys Gly Val Asp Gln Glu Pro Lys Gly Ile Phe Arg Ile Asn Glu
               180                 185                 190
   Asn Thr Gly Ser Val Ser Val Thr Arg Thr Leu Asp Arg Glu Val Ile
           195                 200                 205
   Ala Val Tyr Gln Leu Phe Val Glu Thr Thr Asp Val Asn Gly Lys Thr
       210                 215                 220
   Leu Glu Gly Pro Val Pro Leu Glu Val Ile Val Ile Asp Gln Asn Asp
   225                 230                 235                 240
   Asn Arg Pro Ile Phe Arg Glu Gly Pro Tyr Ile Gly His Val Met Glu
                   245                 250                 255
   Gly Ser Pro Thr Gly Thr Thr Val Met Arg Met Thr Ala Phe Asp Ala
               260                 265                 270
   Asp Asp Pro Ala Thr Asp Asn Ala Leu Leu Arg Tyr Asn Ile Arg Gln
           275                 280                 285
   Gln Thr Pro Asp Lys Pro Ser Pro Asn Met Phe Tyr Ile Asp Pro Glu
       290                 295                 300
   Lys Gly Asp Ile Val Thr Val Val Ser Pro Ala Leu Leu Asp Arg Glu
   305                 310                 315                 320
   Thr Leu Glu Asn Pro Lys Tyr Glu Leu Ile Ile Glu Ala Gln Asp Met
                   325                 330                 335
   Ala Gly Leu Asp Val Gly Leu Thr Gly Thr Ala Thr Ala Thr Ile Met
               340                 345                 350
   Ile Asp Asp Lys Asn Asp His Ser Pro Lys Phe Thr Lys Lys Glu Phe
           355                 360                 365
   Gln Ala Thr Val Glu Glu Gly Ala Val Gly Val Ile Val Asn Leu Thr
       370                 375                 380
   Val Glu Asp Lys Asp Asp Pro Thr Thr Gly Ala Trp Arg Ala Ala Tyr
   385                 390                 395                 400
   Thr Ile Ile Asn Gly Asn Pro Gly Gln Ser Phe Glu Ile His Thr Asn
                   405                 410                 415
   Pro Gln Thr Asn Glu Gly Met Leu Ser Val Val Lys Pro Leu Asp Tyr
               420                 425                 430
   Glu Ile Ser Ala Phe His Thr Leu Leu Ile Lys Val Glu Asn Glu Asp
           435                 440                 445
   Pro Leu Val Pro Asp Val Ser Tyr Gly Pro Ser Ser Thr Ala Thr Val
       450                 455                 460
   His Ile Thr Val Leu Asp Val Asn Glu Gly Pro Val Phe Tyr Pro Asp
   465                 470                 475                 480
   Pro Met Met Val Thr Arg Gln Glu Asp Leu Ser Val Gly Ser Val Leu
                   485                 490                 495
   Leu Thr Val Asn Ala Thr Asp Pro Asp Ser Leu Gln His Gln Thr Ile
               500                 505                 510
   Arg Tyr Ser Val Tyr Lys Asp Pro Ala Gly Trp Leu Asn Ile Asn Pro
           515                 520                 525
   Ile Asn Gly Thr Val Asp Thr Thr Ala Val Leu Asp Arg Glu Ser Pro
       530                 535                 540
   Phe Val Asp Asn Ser Val Tyr Thr Ala Leu Phe Leu Ala Ile Asp Ser
   545                 550                 555                 560
   Gly Asn Pro Pro Ala Thr Gly Thr Gly Thr Leu Leu Ile Thr Leu Glu
                   565                 570                 575
   Asp Val Asn Asp Asn Ala Pro Phe Ile Tyr Pro Thr Val Ala Glu Val
               580                 585                 590
   Cys Asp Asp Ala Lys Asn Leu Ser Val Val Ile Leu Gly Ala Ser Asp
           595                 600                 605
   Lys Asp Leu His Pro Asn Thr Asp Pro Phe Lys Phe Glu Ile His Lys
       610                 615                 620
   Gln Ala Val Pro Asp Lys Val Trp Lys Ile Ser Lys Ile Asn Asn Thr
   625                 630                 635                 640
   His Ala Leu Val Ser Leu Leu Gln Asn Leu Asn Lys Ala Asn Tyr Asn
                   645                 650                 655
   Leu Pro Ile Met Val Thr Asp Ser Gly Lys Pro Pro Met Thr Asn Ile
               660                 665                 670
   Thr Asp Leu Arg Val Gln Val Cys Ser Cys Arg Asn Ser Lys Val Asp
           675                 680                 685
   Cys Asn Ala Ala Gly Ala Leu Arg Phe Ser Leu Pro Ser Val Leu Leu
   690 695 700
   Leu Ser Leu Phe Ser Leu Ala Cys Leu
   705 710
<210> 2
   <211> 244
   <212> PRT
   <213> Homo sapiens
<220>
   <221> SIGNAL
   <222> (1)..(14)
   <223>
<220>
   <221> DOMAIN
   <222> (42) .. (107)
   <223> Collagen-like domain
<220>
   <221> DOMAIN
   <222> (108)..(244)
   <223> Clq domain
<400> 2
   Met Leu Leu Leu Gly Ala Val Leu Leu Leu Leu Ala Leu Pro Gly His
   1 5 10 15
   Asp Gln Glu Thr Thr Thr Gln Gly Pro Gly Val Leu Leu Pro Leu Pro
   20 25 30
   Lys Gly Ala Cys Thr Gly Trp Met Ala Gly Ile Pro Gly His Pro Gly
   35 40 45
   His Asn Gly Ala Pro Gly Arg Asp Gly Arg Asp Gly Thr Pro Gly Glu
   50 55 60
   Lys Gly Glu Lys Gly Asp Pro Gly Leu Ile Gly Pro Lys Gly Asp Ile
   65 70 75 80
   Gly Glu Thr Gly Val Pro Gly Ala Glu Gly Pro Arg Gly Phe Pro Gly
   85 90 95
   Ile Gln Gly Arg Lys Gly Glu Pro Gly Glu Gly Ala Tyr Val Tyr Arg
   100 105 110
   Ser Ala Phe Ser Val Gly Leu Glu Thr Tyr Val Thr Ile Pro Asn Met
   115 120 125
   Pro Ile Arg Phe Thr Lys Ile Phe Tyr Asn Gln Gln Asn His Tyr Asp
   130 135 140
   Gly Ser Thr Gly Lys Phe His Cys Asn Ile Pro Gly Leu Tyr Tyr Phe
   145 150 155 160
   Ala Tyr His Ile Thr Val Tyr Met Lys Asp Val Lys Val Ser Leu Phe
   165 170 175
   Lys Lys Asp Lys Ala Met Leu Phe Thr Tyr Asp Gln Tyr Gln Glu Asn
   180 185 190
   Asn Val Asp Gln Ala Ser Gly Ser Val Leu Leu His Leu Glu Val Gly
   195 200 205
   Asp Gln Val Trp Leu Gln Val Tyr Gly Glu Gly Glu Arg Asn Gly Leu
   210 215 220
   Tyr Ala Asp Asn Asp Asn Asp Ser Thr Phe Thr Gly Phe Leu Leu Tyr
   225 230 235 240
   His Asp Thr Asn
<210> 3
   <211> 247
   <212> PRT
   <213> Mus musculus
<220>
   <221> SIGNAL
   <222> (1)..(17)
   <223>
<220>
   <221> DOMAIN
   <222> (45) .. (110)
   <223> Collagen-like domain
<220>
   <221> DOMAIN
   <222> (111)..(247)
   <223> C1q domain
<400> 3
   Met Leu Leu Leu Gln Ala Leu Leu Phe Leu Leu Ile Leu Pro Ser His
   1               5                   10                  15
   Ala Glu Asp Asp Val Thr Thr Thr Glu Glu Leu Ala Pro Ala Leu Val
               20                  25                  30
   Pro Pro Pro Lys Gly Thr Cys Ala Gly Trp Met Ala Gly Ile Pro Gly
           35                  40                  45
   His Pro Gly His Asn Gly Thr Pro Gly Arg Asp Gly Arg Asp Gly Thr
       50                  55                  60
   Pro Gly Glu Lys Gly Glu Lys Gly Asp Ala Gly Leu Leu Gly Pro Lys
   65                  70                  75                  80
   Gly Glu Thr Gly Asp Val Gly Met Thr Gly Ala Glu Gly Pro Arg Gly
                   85                  90                  95
   Phe Pro Gly Thr Pro Gly Arg Lys Gly Glu Pro Gly Glu Ala Ala Tyr
               100                 105                 110
   Met Tyr Arg Ser Ala Phe Ser Val Gly Leu Glu Thr Arg Val Thr Val
           115                 120                 125
   Pro Asn Val Pro Ile Arg Phe Thr Lys Ile Phe Tyr Asn Gln Gln Asn
       130                 135                 140
   His Tyr Asp Gly Ser Thr Gly Lys Phe Tyr Cys Asn Ile Pro Gly Leu
   145                 150                 155                 160
   Tyr Tyr Phe Ser Tyr His Ile Thr Val Tyr Met Lys Asp Val Lys Val
                   165                 170                 175
   Ser Leu Phe Lys Lys Asp Lys Ala Val Leu Phe Thr Tyr Asp Gln Tyr
               180                 185                 190
   Gln Glu Lys Asn Val Asp Gln Ala Ser Gly Ser Val Leu Leu His Leu
           195                 200                 205
   Glu Val Gly Asp Gln Val Trp Leu Gln Val Tyr Gly Asp Gly Asp His
       210                 215                 220
   Asn Gly Leu Tyr Ala Asp Asn Val Asn Asp Ser Thr Phe Thr Gly Phe
   225                 230                 235                 240
   Leu Leu Tyr His Asp Thr Asn
                   245
<210> 4
   <211> 714
   <212> PRT
   <213> Mus musculus
<220>
   <221> SIGNAL
   <222> (1)..(22)
   <223>
<220>
   <221> LIPID
   <222> (693)..(693)
   <223> GPI-ANCHOR
<220>
   <221> PROPEP
   <222> (23) .. (139)
   <223>
<220>
   <221> PROPEP
   <222> (694)..(714)
   <223>
<400> 4
   Met Gln Pro Arg Thr Pro Leu Thr Leu Cys Val Leu Leu Ser Gln Val
   1 5 10 15
   Leu Leu Val Thr Ser Ala Asp Asp Leu Glu Cys Thr Pro Gly Phe Gln
   20 25 30
   Arg Lys Val Leu His Ile His Gln Pro Ala Glu Phe Ile Glu Asp Gln
   35 40 45
   Pro Val Leu Asn Leu Thr Phe Asn Asp Cys Lys Gly Asn Glu Lys Leu
   50 55 60
   His Tyr Glu Val Ser Ser Pro His Phe Lys Val Asn Ser Asp Gly Thr
   65 70 75 80
   Leu Val Ala Leu Arg Asn Ile Thr Ala Val Gly Arg Thr Leu Phe Val
   85 90 95
   His Ala Arg Thr Pro His Ala Glu Asp Met Ala Glu Leu Val Ile Val
   100 105 110
   Gly Gly Lys Asp Ile Gln Gly Ser Leu Gln Asp Ile Phe Lys Phe Ala
   115 120 125
   Arg Thr Ser Pro Val Pro Arg Gln Lys Arg Ser Ile Val Val Ser Pro
   130 135 140
   Ile Leu Ile Pro Glu Asn Gln Arg Gln Pro Phe Pro Arg Asp Val Gly
   145 150 155 160
   Lys Val Val Asp Ser Asp Arg Pro Glu Gly Ser Lys Phe Arg Leu Thr
   165 170 175
   Gly Lys Gly Val Asp Gln Asp Pro Lys Gly Thr Phe Arg Ile Asn Glu
   180 185 190
   Asn Thr Gly Ser Val Ser Val Thr Arg Thr Leu Asp Arg Glu Thr Ile
   195 200 205
   Ala Thr Tyr Gln Leu Tyr Val Glu Thr Thr Asp Ala Ser Gly Lys Thr
   210 215 220
   Leu Glu Gly Pro Val Pro Leu Glu Val Ile Val Ile Asp Gln Asn Asp
   225 230 235 240
   Asn Arg Pro Ile Phe Arg Glu Gly Pro Tyr Ile Gly His Val Met Glu
   245 250 255
   Gly Ser Pro Thr Gly Thr Thr Val Met Arg Met Thr Ala Phe Asp Ala
   260 265 270
   Asp Asp Pro Ala Thr Asp Asn Ala Leu Trp Arg Tyr Asn Ile Arg Gln
   275 280 285
   Gln Thr Pro Asp Lys Pro Ser Pro Asn Met Phe Tyr Ile Asp Pro Glu
   290 295 300
   Lys Gly Asp Ile Val Thr Val Val Ser Pro Ala Leu Leu Asp Arg Glu
   305                 310                 315                 320
   Thr Leu Glu Asn Pro Lys Tyr Glu Leu Ile Ile Glu Ala Gln Asp Met
                   325                 330                 335
   Ala Gly Leu Asp Val Gly Leu Thr Gly Thr Ala Thr Ala Thr Ile Val
               340                 345                 350
   Ile Asp Asp Lys Asn Asp His Ser Pro Lys Phe Thr Lys Lys Glu Phe
           355                 360                 365
   Gln Ala Arg Val Glu Glu Gly Ala Val Gly Val Ile Val Asn Leu Thr
       370                 375                 380
   Val Glu Asp Lys Asp Asp Pro Thr Thr Gly Ala Trp Arg Ala Ala Tyr
   385                 390                 395                 400
   Thr Ile Ile Asn Gly Asn Pro Gly Gln Ser Phe Glu Ile His Thr Asn
                   405                 410                 415
   Pro Gln Thr Asn Glu Gly Met Leu Ser Val Val Lys Pro Leu Asp Tyr
               420                 425                 430
   Glu Ile Ser Ala Phe His Thr Leu Leu Ile Lys Val Glu Asn Glu Asp
           435                 440                 445
   Pro Leu Val Pro Asp Val Ser Tyr Gly Pro Ser Ser Thr Ala Thr Val
       450                 455                 460
   His Ile Thr Val Leu Asp Val Asn Glu Gly Pro Val Phe Tyr Pro Asp
   465                 470                 475                 480
   Pro Met Met Val Thr Lys Gln Glu Asn Ile Ser Val Gly Ser Val Leu
                   485                 490                 495
   Leu Thr Val Asn Ala Thr Asp Pro Asp Ser Leu Gln His Gln Thr Ile
               500                 505                 510
   Arg Tyr Ser Ile Tyr Lys Asp Pro Ala Gly Trp Leu Ser Ile Asn Pro
           515                 520                 525
   Ile Asn Gly Thr Val Asp Thr Thr Ala Val Leu Asp Arg Glu Ser Pro
       530                 535                 540
   Phe Val His Asn Ser Val Tyr Thr Ala Leu Phe Leu Ala Ile Asp Ser
   545                 550                 555                 560
   Gly Asn Pro Pro Ala Thr Gly Thr Gly Thr Leu Leu Ile Thr Leu Glu
                   565                 570                 575
   Asp Ile Asn Asp Asn Ala Pro Val Ile Tyr Pro Thr Val Ala Glu Val
               580                 585                 590
   Cys Asp Asp Ala Arg Asn Leu Ser Val Val Ile Leu Gly Ala Ser Asp
           595                 600                 605
   Lys Asp Leu His Pro Asn Thr Asp Pro Phe Lys Phe Glu Ile His Lys
       610                 615                 620
   Gln Thr Val Pro Asp Lys Val Trp Lys Ile Ser Lys Ile Asn Asn Thr
   625                 630                 635                 640
   His Ala Leu Val Ser Leu Leu Gln Asn Leu Asn Lys Ala Asn Tyr Asn
                   645                 650                 655
   Leu Pro Ile Met Val Thr Asp Ser Gly Lys Pro Pro Met Thr Asn Ile
               660                 665                 670
   Thr Asp Leu Lys Val Gln Val Cys Ser Cys Lys Asn Ser Lys Val Asp
           675                 680                 685
   Cys Asn Gly Ala Gly Ala Leu His Leu Ser Leu Ser Leu Leu Leu Leu
       690                 695                 700
   Phe Ser Leu Leu Ser Leu Leu Ser Gly Leu
   705                 710
<210> 5
   <211> 36
   <212> DNA
   <213> Artificial
<220>
   <223> primer
<400> 5
   aagaattccg ccaccatgct actgttgcaa gctctc 36
<210> 6
   <211> 45
   <212> DNA
   <213> Artificial
<220>
   <223> primer
<400> 6
   gactacaagg acgacgatga caaggaagat gacgttacta caact 45
<210> 7
   <211> 45
   <212> DNA
   <213> Artificial
<220>
   <223> primer
<400> 7
   cttgtcatcg tcgtccttgt agtcggcatg actgggcagg attaa 45
<210> 8
   <211> 30
   <212> DNA
   <213> Artificial
<220>
   <223> primer
<400>
   tttgaattct cagttggtat catggtagag 30
<210> 9
   <211> 36
   <212> DNA
   <213> Artificial
<220>
   <223> primer
<400> 9
   aagaattccg ccaccatgct actgttgcaa gctctc 36
<210> 10
   <211> 60
   <212> DNA
   <213> Artificial
<220>
   <223> primer
<400> 10
   tttgaattct cacttgtcgt catcgtcttt gtagtctgca cttgcatcgt tggtatcatg 60
<210> 11
   <211> 18
   <212> DNA
   <213> Artificial
<220>
   <223> primer
<400> 11
   gacatctcct gtcccaag 18
<210> 12
   <211> 18
   <212> DNA
   <213> Artificial
<220>
   <223> primer
<400> 12
   ctaacatgtt ctacatcg 18
<210> 13
   <211> 18
   <212> DNA
   <213> Artificial
<220>
   <223> primer
<400> 13
   ctgtccacat cacagtcc 18
<210> 14
   <211> 19
   <212> DNA
   <213> Artificial
<220>
   <223> primer
<400> 14
   cagacagtcc ctgataaag 19
<210> 15
   <211> 20
   <212> DNA
   <213> Artificial
<220>
   <223> primer
<400> 15
   ctcgttgccc ttgcagtcac 20
<210> 16
   <211> 19
   <212> DNA
   <213> Artificial
<220>
   <223> primer
<400> 16
   gacttccaga ggcactggc 19
<210> 17
   <211> 19
   <212> DNA
   <213> Artificial
<220>
   <223> primer
<400> 17
   ggctcctgtg gtggggtcg 19
<210> 18
   <211> 18
   <212> DNA
   <213> Artificial
<220>
   <223> primer
<400> 18
   ggttgccact gtcgatgg 18